(19) Europäisches Patentamt
European Patent Office
Office européen des brevets

(11) Publication number: **0 404 267 A1**

(12) # EUROPEAN PATENT APPLICATION

(21) Application number: **90201621.1**

(22) Date of filing: **20.06.90**

(51) Int. Cl.5: **C07D 205/085, C07D 401/04, C07D 409/04, C07D 405/04, C07F 7/10, C07F 3/00, C07C 251/08, C07D 213/56**

(30) Priority: **22.06.89 EP 89201666**
**12.12.89 EP 89203168**

(43) Date of publication of application:
**27.12.90 Bulletin 90/52**

(84) Designated Contracting States:
**AT BE CH DE DK ES FR GB GR IT LI LU NL SE**

(71) Applicant: **RIJKSUNIVERSITEIT UTRECHT**
**Padualaan 8**
**NL-3584 CH Utrecht(NL)**

(72) Inventor: **van Koten, Gerard**
**Paltzerweg 180**
**NL-3734 CN Den dolder(NL)**
Inventor: **van der Steen, Frederik Hendrik**
**Huetlaan 11**
**NL-3553 GN Utrecht(NL)**
Inventor: **Jastrzebski, Johann Töns Barthold Heinrich**
**Stratusplein 11**
**NL-3731 XA De Bilt(NL)**
Inventor: **Kleijn, Henk**
**Aquamarijndrift 9**
**NL-3436 BD Nieuwegein(NL)**
Inventor: **Britovsek, George**
**Broekhuizenstraat 103**
**NL-6374 LK Landgraaf(NL)**

(74) Representative: **Matulewicz, Emil Rudolf Antonius, Dr. et al**
**Gist-Brocades NV Patents and Trademarks Department Wateringseweg 1 P.O. Box 1**
**NL-2600 MA Delft(NL)**

(54) **Improved synthesis of beta-lactams using a metal compound.**

(57) A synthesis of trans-β-lactams with an easily convertible group on the C-4 carbon atom of the azetidinone ring comprising a condensation reaction of a metal enolate with an imine with a heterocyclic group or a diimine is provided.

Furthermore, new chiral β-lactams and an enantioselective synthesis of β-lactams using a chiral imine or α-diimine are provided. Also new chiral substituted amino propionic ester metal compounds and new chiral amino acid compounds and processes for the preparation thereof are provided.

EP 0 404 267 A1

## IMPROVED SYNTHESIS OF β-LACTAMS USING A METAL COMPOUND

The present invention relates to the synthesis of trans-β-lactams, to new chiral trans-β-lactams and to the enantio-selective synthesis of such chiral compounds.

Much research effort has been devoted to the development of new methods of synthesizing β-lactams, which compounds are potent antibiotics.

In EP-A-281177 a synthetic route is described to 1,3,4-trisubstituted trans-2-azetidinones, in which the atom numbering conventionally is as follows:

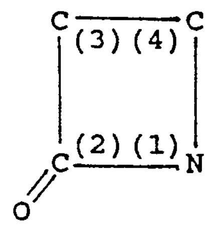

A process starting from a metal enolate (II′), an alkali metal base (P-base) and a metal compound (VIII′), and a one-pot process starting from an $R_2'''R_3'''$N-glycine ester (V′), both involving reaction with an imine (IV′), are described in EP-A-281177 (see the following scheme I):

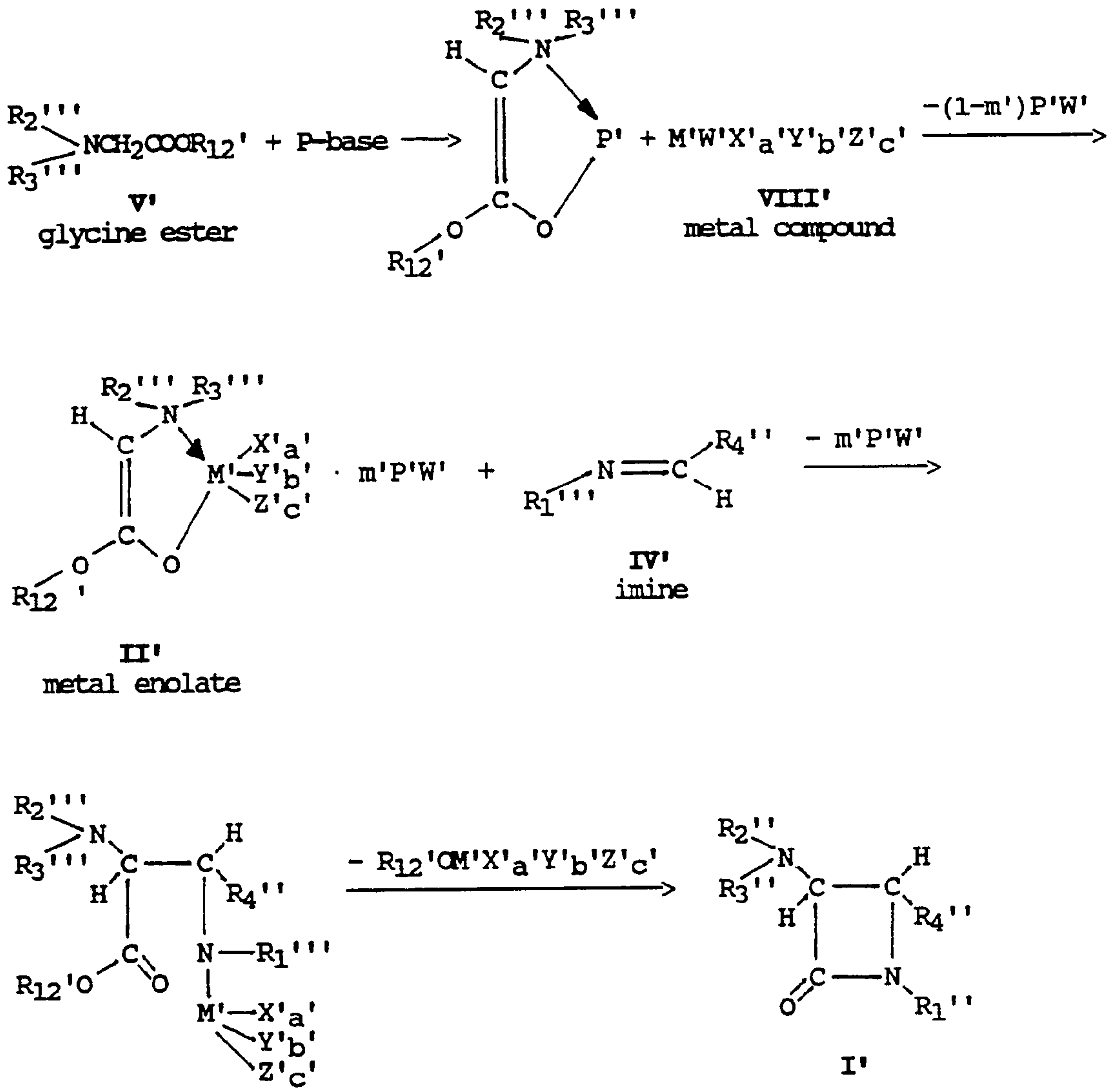


V' glycine ester

VIII' metal compound

II' metal enolate

IV' imine

I'

**Scheme I**

wherein
$R2''$ and $R3''$ are each, independently, hydrogen, alkyl, aryl or aralkyl, each optionally substituted by an alkyl, aryl or aralkyl group, with the proviso that they are not both methyl or both benzyl; or are

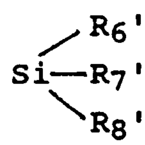

where $R_6'$, $R_7'$ and $R_8'$ are each alkyl, aryl or aralkyl, each optionally substituted and $R_6'$, $R_7'$ and $R_8'$ are the same or different;
or $R_2''$ and $R_3''$, together with the nitrogen atom to which they are attached, form a ring with up to 8 ring atoms, each ring atom optionally substituted by an alkyl, aryl or aralkyl group;
$R_1''$ is hydrogen, halogen, or an alkyl, alkenyl, alkoxy, aryl or aralkyl group, or is

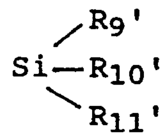

where $R_9'$, $R_{10}'$ and $R_{11}'$ are each, independently, an alkyl, aryl or aralkyl group, each optionally substituted;
$R_4''$ is an alkyl, hydroxy, halogen, sulfonyl, alkoxy, alkenyl, alkynyl or aryl group, each optionally substituted, or is

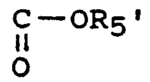

where $R_5'$ is an alkyl group;
$R_{12}'$ is an alkyl, aryl or aralkyl group, each optionally substituted by an alkyl, aryl or aralkyl group;
$M'$ is zinc, aluminum, zirconium, boron, tin or titanium;
$P'$ is an alkali metal;
$W'$, $X'$, $Y'$ and $Z'$ are each, independently, an alkyl, aryl, halide, alkoxide, thiolate, triflate or other substituted sulfonate or other anionic group;
and $a'$, $b'$, $c'$ and $m'$ are each from 0 to 1 with $a'$, $b'$, $c'$ being integers;
$R_2'''$ and $R_3'''$ are as defined above for $R_2''$ and $R_3''$, respectively, with the proviso that when $R_2''$ and $R_3''$ are hydrogen, $R_2'''$ and $R_3'''$ form, together with the nitrogen atom to which they are attached, a ring of formula III'

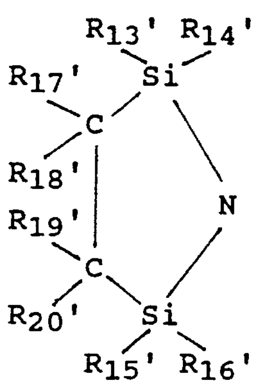

III'

wherein
$R_{13}'$, $R_{14}'$, $R_{15}'$, $R_{16}'$, $R_{17}'$, $R_{18}'$, $R_{19}'$ and $R_{20}'$ are each, independently, an alkyl, aryl or aralkyl group, which group is subjected to acid or base hydrolysis after the reaction;
$R_1'''$ is as defined above for $R_1''$, with the proviso that when $R_1''$ is hydrogen, $R_1'''$ is

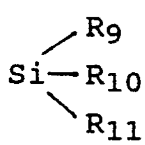

where $R_9$, $R_{10}$ and $R_{11}$ are as defined above,
which group is subjected to acid or base hydrolysis after the reaction.

The processes described in EP-A-281177, which both start with the metal enolate and with the $R_2'R_3'$N-glycine ester and the metal compound, respectively, allow trans-$\beta$-lactams (trans-azetidinones) with 1-H (- or hydrolysable group), and 3-$NH_2$ substituents to be synthesized. However, the described synthesis does not yield an easily convertible group linked to the C-4 carbon atom. Such a group in this position is desirable because it yields an intermediate for a process to synthesize (carba)cephems or (carba)penems, which are antibiotics.

Furthermore, the synthetic routes described in EP-A-281177 involve enolates which yield trans-β-lactams, cis-β-lactams or a mixture thereof. However, these syntheses are a-specific and do not result in one enantiomer of a trans-β-lactam or a cis-β-lactam. The enantiomeric forms of the trans-β-lactam refer to the configuration of the groups linked to the C-3 carbon atom and to the C-4 carbon atom. The synthesis of a pure enantiomer of a trans-β-lactam is important because such enantiomers are intermediates to (carba)-cephems and (carba)penems. These latter compounds are only active with a specific configuration at the C-3 and C-4 carbon atoms.

It has now surprisingly been found that the reactions disclosed in EP-A-281177 may still be carried out if, as the imine of formula IV', an α-diimine compound or an imine of which the carbon is substituted by an optionally substituted heterocyclic group, is used. In the resulting trans-azetidinones corresponding to formula I', R4'' is then either an imine substituent or an optionally substituted heterocyclic group. When R4'' is an imine substituent, it would have been expected that a double condensation reaction, resulting in two coupled azetidinone rings, would occur. Surprisingly, however, a trans-azetidinone corresponding to formula I' forms instead.

The present invention accordingly provides a process for the preparation of a trans-β-lactam compound of formula I,

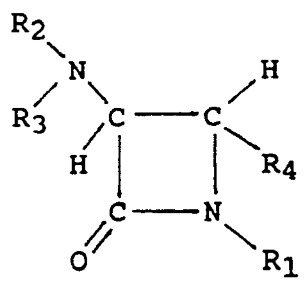

I

wherein

$R_2$ and $R_3$ are each, independently, hydrogen, or a (1-8C)alkyl, (6-18C)aryl or (1-8C)alkyl(6-18C)aryl group, each optionally substituted by a (1-8C)alkyl, (6-18C)aryl or (1-8C)alkyl(6-18C)aryl group, or the group

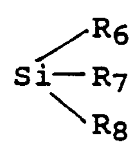

where $R_6$, $R_7$ and $R_8$ are each, independently, an optionally substituted (1-8C)alkyl, (6-18C)aryl or (1-8C)-alkyl(6-18C)aryl group,

or $R_2$ and $R_3$, together with the nitrogen atom to which they are attached, form a ring with up to 8 ring atoms, each ring atom being optionally substituted by a (1-8C)alkyl, (6-18C)aryl or (1-8C)alkyl(6-18C)aryl group;

$R_1$ is hydrogen, halogen, a (1-8C)alkyl, (2-8C)alkenyl, (1-8C)alkoxy, (6-18C)aryl, (6-18C)aryloxy, (1-8C)alkyl-(6-18C)aryl, (1-8C)alkoxy(6-18C)aryl or heterocyclic group, or is the group

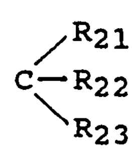

where $R_{21}$, $R_{22}$ and $R_{23}$ are each, independently, hydrogen or an optionally substituted (1-8C)alkyl, (1-8C)-alkoxy, (6-18C)aryl, (6-18C)aryloxy, (1-8C)alkyl(6-18C)aryl, (1-8C)alkoxy(6-18C)aryl, heterocyclic or

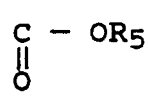

group

with $R_5$ is an (1-8C)alkyl, (6-18C)aryl or (1-8C)alkyl(6-18C)aryl group, or is the group

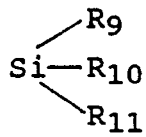

where $R_9$, $R_{10}$ and $R_{11}$ are each, independently, an optionally substituted (1-8C)alkyl, (6-18C)aryl or (1-8C)-alkyl(6-18C)aryl group;

$R_4$ is 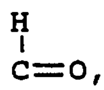, or 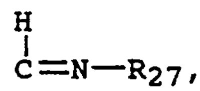,

where $R_{27}$ is an optionally substituted (1-8C)alkyl, (6-18C)aryl, (1-8C)alkyl(6-18C)aryl or heterocyclic group, or

$R_{27}$ is 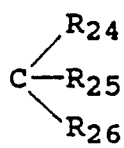

where $R_{24}$, $R_{25}$ and $R_{26}$ are each, independently, hydrogen or an optionally substituted (1-8C)alkyl, (1-8C)-alkoxy, (6-18C)aryl, (6-18C)aryloxy, (1-8C)alkyl(6-18C)aryl, (1-8C)alkoxy(6-18C)aryl or heterocyclic group, or $R_4$ is an optionally substituted heterocyclic group, which process comprises reacting a compound of formula II,

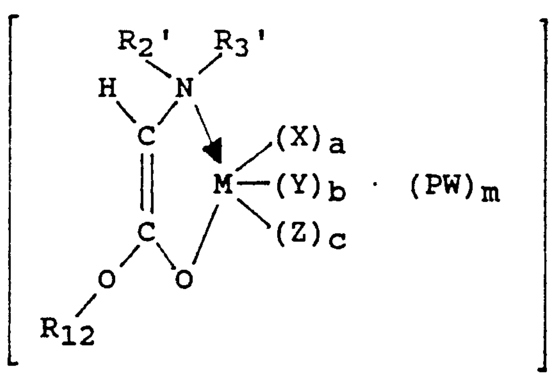

II

wherein

$R_{12}$ is a (1-8C)alkyl, (4-8C)cycloalkyl, (6-18C)aryl or (1-8C)alkyl(6-18C)aryl group, each optionally substituted by a (1-8C)alkyl, (4-8C)cycloalkyl, (6-18C)aryl or (1-8C)alkyl(6-18C)aryl group;
M is zinc, aluminum, zirconium, boron, tin or titanium;
P is an alkali metal;
X, Y and Z are each, independently, a (1-8C)alkyl or (6-18C)aryl group, or an anionic group;
W is an anionic group;
and a, b, c and m are each, independently, from 0 to 1 with the proviso that one of a, b or c is 1;
n is from 1 to 6, with a, b, c and n being integers;
$R_2'$ and $R_3'$ are as defined for $R_2$ and $R_3$, respectively, with the proviso that when $R_2$ and $R_3$ are hydrogen, $R_2'$ and $R_3'$ form, together with the nitrogen atom to which they are attached, a ring of formula III

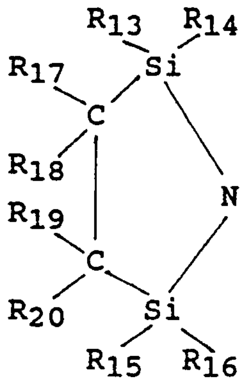

III

wherein
$R_{13}$, $R_{14}$, $R_{15}$, $R_{16}$, $R_{17}$, $R_{18}$, $R_{19}$ and $R_{20}$ are each, independently, a (1-8C)alkyl, (6-18C)aryl or (1-8C)-alkyl(6-18C)aryl group, and $R_{17}$, $R_{18}$, $R_{19}$ and $R_{20}$ are each, independently, hydrogen,
with an imine of formula IV,

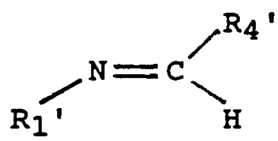

IV

where $R_1'$ is as defined for $R_1$, with the proviso that when $R_1$ is hydrogen, $R_1'$ is

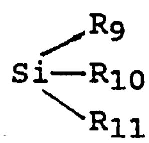

where $R_9$, $R_{10}$ and $R_{11}$ are as defined above,
which group is subjected, after the reaction of compounds II and IV, to acid or base hydrolysis,
or $R_1'$ is

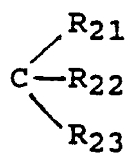

where $R_{21}$, $R_{22}$ and $R_{23}$ are as defined above,
which group is subjected to the additional reaction with a solution of an alkali metal, such as sodium or lithium, in a mixture of ammonia and a solvent, for instance tetrahydrofuran,
and $R_4'$ is as defined for $R_4$, with the proviso that when

$R_4$ is 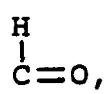, $R_4'$ is 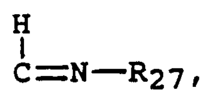,

which group is subjected, after the reaction of compounds II and IV, to acid hydrolysis, or
$R_4'$ is (1,2,0-isopropylidene)ethyl, which group is subjected, after the reaction of compounds II and IV, to oxidation, for instance with sodium iodate or lead tetraacetate,
and with the additional proviso that when $R_2$ and $R_3$ are both hydrogen, the process includes the additional step of converting the ring of formula III into $NH_2$ by acid or base catalysed hydrolysis.

Particularly, W is selected from a halide, thiolate and substituted sulfonate group and X, Y and Z are

each, independently, an anionic group selected from halide, alkoxide, thiolate and substituted sulfonate such as triflate.

It has further been found that the preparation of trans-β-lactam compounds of formula I also can be carried out as a one-pot process which comprises reacting a glycine ester of formula V

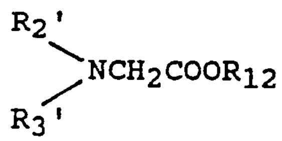

V

wherein
$R_2'$, $R_3'$ and $R_{12}$ are as defined above,
with an imine of formula IV as defined above, in the presence of an alkali metal base and a metal compound of formula $MW(X)_a(Y)_b(Z)_c$, wherein M, W, X, Y, Z, a, b and c are as defined above. This one-pot process forms a further aspect of the invention.

It has also been surprisingly found that the use of a chiral imine or chiral α-diimine allows new chiral β-lactams to be prepared with a very high enantioselectivity.

The formation of a chiral trans- or cis-β-lactam is dependent on the solvent used. A trans-β-lactam is formed in a solvent suitable for the formation thereof, generally a (weakly) polar solvent such as diethyl ether. Also a cis-β-lactam compound has been found to be formed in a solvent suitable for the formation thereof, generally in a polar solvent such as a mixture of hexamethyl phosphoric triamide and tetrahydrofuran.

Of course, also following these reactions involving a chiral group, the imine group may be hydrolysed into the aldehyde group. A chiral trans-β-lactam with $R_4$ is aldehyde can easily be converted into a chiral (carba)penem or (carba)cephem. A chiral imine or chiral α-diimine as used herein means an imine or α-diimine with at least one chiral group.

Accordingly, the present invention further provides chiral trans-β-lactam compounds of formula Ia and of formula Ib

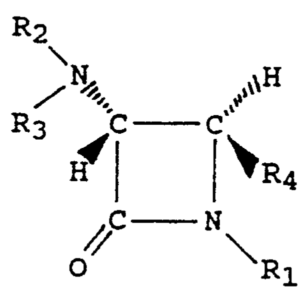

Ia

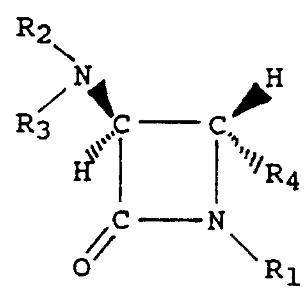

Ib

and chiral cis-β-lactams of formula Ic and Id

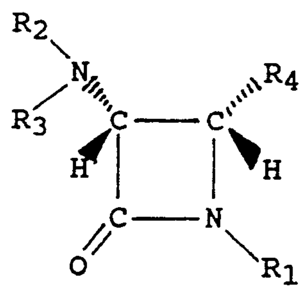

Ic

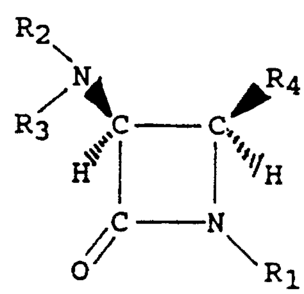

Id

wherein
$R_1$, $R_2$ and $R_3$ are as defined above or

$R_2$ and $R_3$ form, together with the nitrogen atom to which they are attached, a carbamate group; and
$R_4$ is as defined above,
or is (1-8C)alkyl, (1-8C)alkoxy, (6-18C)aryl, (6-18C)aryloxy, (1-8C)alkyl(6-18C)aryl, (1-8C)alkoxy(6-18C)aryl, hydroxy, sulfonyl, (2-8C)alkenyl, (2-8C)alkynyl, each optionally substituted,
or is

$$-\underset{\underset{O}{\|}}{C}-OR_5,$$

where $R_5$ is an (1-8C)alkyl, (6-18C)aryl or (1-8C)alkyl(6-18C)aryl group, each optionally substituted, with the proviso that either $R_1$ or $R_4$ comprises a chiral group, or $R_1$ is hydrogen or

$$R_4 \text{ is } \overset{\overset{O}{\|}}{C}-H.$$

As indicated above, compounds of formula Ia and Ib were found to be prepared by the process, or the one-pot process, described above for the preparation of compounds of formula I, but using a suitable chiral imine or chiral $\alpha$-diimine of formula IV as defined above, with the proviso that when $R_2$ and $R_3$ are hydrogen, $R_2'$ and $R_3'$ can also form a carbamate group and that the process then includes the additional step of converting the carbamate group into $NH_2$ by base catalysed hydrolysis,
in a suitable, generally weakly polar solvent, such as diethyl ether. This enantioselective preparation constitutes a further aspect of the present invention.

As indicated above, compounds of formula Ic and Id were found to be prepared by the process, or the one-pot process, described above for the preparation of compounds of formula Ia and Ib, but in a suitable polar solvent, such as a mixture of hexamethyl phosphoric triamide and tetrahydrofuran. This enantioselective preparation constitutes a further aspect of the present invention.

The chiral $\beta$-lactams described above are novel, with the exception of $R_4$ being (1-8C)alkoxy, (6-18C)-aryl, (6-18C)aryloxy, (1-8C)alkyl(6-18C)aryl, (1-8C)alkoxy(6-18C)aryl, hydroxy, sulfonyl, (2-8C)alkenyl, (2-8C)alkynyl, each optionally substituted,
or

$$-\underset{\underset{O}{\|}}{C}-OR_5,$$

where $R_5$ is as defined above. The already known compounds and the preparation thereof by a different process, for example starting from L-aspartic acid, are disclosed in, for instance, Chem. Pharm. Bull. 34, 2732-2742 (1986).

It has been found further that, if the reaction of a glycine ester, an alkali metal base, a metal compound and a suitable chiral imine or chiral $\alpha$-diimine of formula IV or as defined above, is conducted in an apolar solvent, for instance benzene, then instead of a $\beta$-lactam a new chiral substituted amino propionic ester is obtained. In a weakly polar or polar solvent this chiral amino propionic ester rearranges to the chiral $\beta$-lactam, as shown in Scheme II:

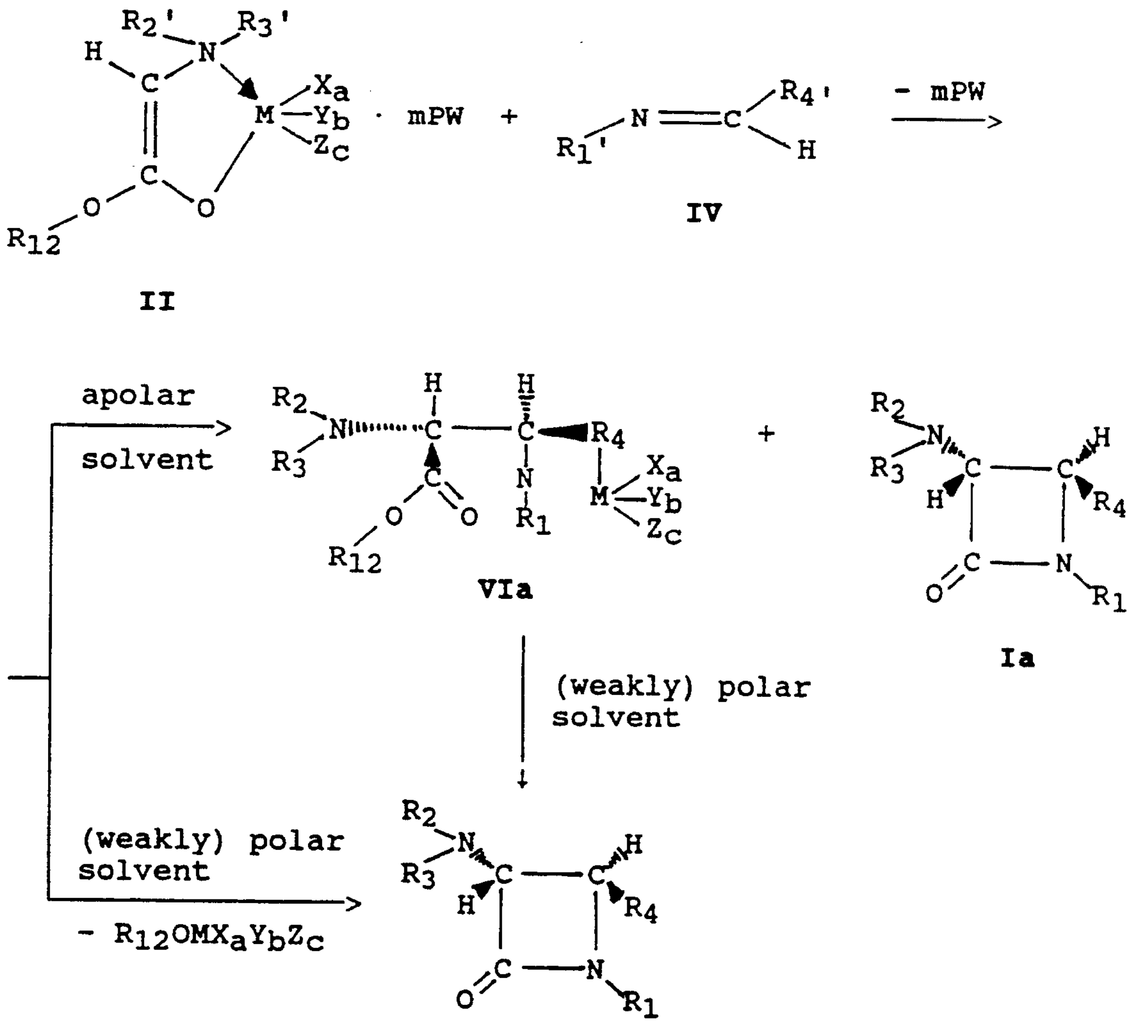


II

IV

VIa

Ia

Ia

**Scheme II**

Accordingly the present invention further provides new chiral substituted amino propionic ester metal compounds of formula VIa and VIb

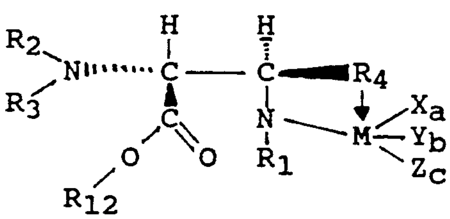

VIa

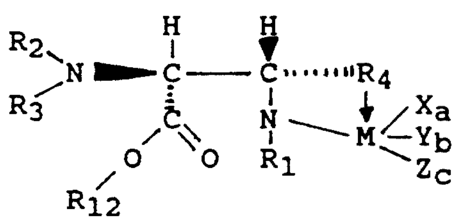

VIb

and of formula VIc and VId

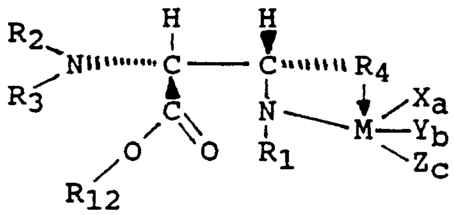

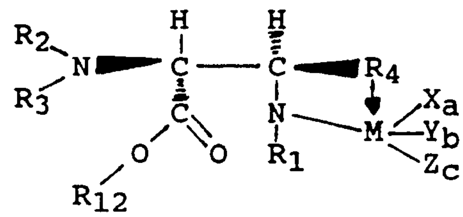

VIc VId

wherein

$R_1$, $R_2$, $R_3$ and $R_4$ for formula VIa and VIb are as defined above for formula Ia, Ib, Ic and 1d, and

$R_{12}$, M, X, Y, Z, a, b, and c are as defined above for formula II.

There is also provided a process for the preparation of compounds of formula VIa and VIb or VIc and VId which comprises reacting, in an apolar solvent, a compound of formula II as defined above with a chiral imine or chiral $\alpha$-diimine of formula IV, as defined above. An example of a suitable apolar solvent is benzene.

The invention also provides a one-pot process for the preparation of a compound of formula VIa and VIb or VIc and VId which comprises reacting, in an apolar solvent, a glycine ester of formula V as defined above with a chiral imine of formula IV, as defined above, in the presence of an alkali metal base and a metal compound with the formula $MW(X)_a(Y)_b(Z)_c$ where M, W, X, Y, Z, a, b and c are as defined above.

The invention also provides a process for the preparation of chiral trans-$\beta$-lactams of formula Ia and Ib, respectively, as defined above, by adding a suitable (weakly) polar solvent to a chiral substituted amino ester metal compound of formula VIa and VIb, respectively, as defined above. Typically the solvent is tetrahydrofuran.

The invention further provides a process for the preparation of chiral cis-$\beta$-lactams of formula Ic and Id, as defined above, by adding a suitable (weakly) polar solvent, such as tetrahydrofuran, to a chiral substituted amino ester metal compound of formula VIc and VId, respectively.

The new chiral $\beta$-lactams of the present invention can be converted by treatment with hydrogen in a conventional manner, into new chiral amino acids, by cleaving the N-$C_4$ bonds, see for instance Scheme III:

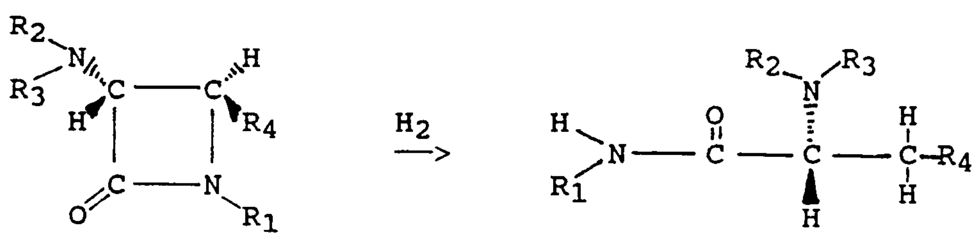

Ia VIIa

**Scheme III**

The invention therefore also provides new amino acid derivatives, with a chiral group, of formula VIIa and VIIb

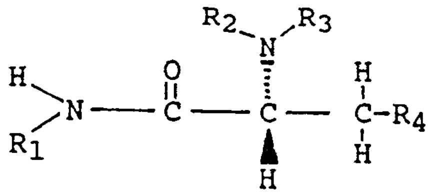

VIIa

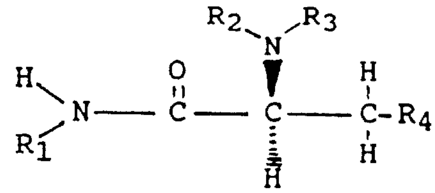

VIIb

wherein
$R_1$, $R_2$, $R_3$ and $R_4$ are as defined above for formula Ia, Ib, Ic or Id,
and a process for the preparation thereof, comprising reacting a suitable chiral trans-β-lactam compound of formula Ia or Ic and Ib or Id, respectively, as defined above, with hydrogen. This reaction is conducted in a conventional manner, for example by treating the lactam with palladium on carbon and dry ammonium formate.

These chiral amino acids can be converted into chiral β-lactams again and are useful as side-chain precursors for β-lactams.

The reactions described above for the preparation of trans-β-lactams of formula I involving an α-diimine or an imine of which the carbon atom is substituted by an optionally substituted heterocyclic group are preferably carried out in inert, (weakly) polar solvents, for instance diethyl ether or tetrahydrofuran.

In the case of reactions involving a chiral imine or a chiral α-diimine where a trans-β-lactam is the desired product, then, as discussed above, a suitable solvent to form a trans-β-lactam compound, generally a weakly polar solvent such as diethyl ether, dimethoxyethane, t-butyl ether or dioxane has found to be used. If on the other hand a cis-β-lactam is the desired product, then, as discussed above, generally a polar solvent such as hexamethyl phosphoric triamide or hexamethyl phosphorous triamide has found to be used. If as solvent tetrahydrofuran, borderline between weakly polar and polar is used, then dependent on the substituents a trans- or cis-β-lactam is formed. If in that case a trans-β-lactam is formed in tetrahydrofuran, then a cis-β-lactam has been found to be formed in a mixture of tetrahydrofuran and hexamethyl phosphoric triamide. All the processes as defined above in all (combinations of) solvents suitable to form either a trans- or cis-β-lactam compound of formula Ia and Ib, or Ic and Id, respectively, form part of this invention.

If a chiral trans- or cis-β-lactam is prepared from a chiral substituted amino ester metal compound, a (weakly) polar solvent is suitable. Because the right chirality already is present in the starting compound, there is no difference in the solvent for the preparation of chiral trans-or cis-β-lactams.

If a chiral amino propionic ester is the desired product, as discussed above, an apolar solvent such as aromatics, particularly benzene, or alkanes, such as hexane, has to be used.

M in the metal compound $MW(X)_a(Y)_b(Z)_c$ is suitably zinc, aluminum, zirconium, boron, tin or titanium. Zinc is particularly suitable and $ZnCl_2$ is a preferred compound.

The term "chiral β-lactam" as used herein means a β-lactam with an absolute configuration at the C-3 and C-4 carbon atoms. It depends on the substituent $R_4$ whether this configuration has to be described as 3R,4S or 3R,4R for formula Ia and Ic, or as 3S,4R or 3S,4S for formula Ib and Id.

The chiral imine or chiral α-diimine bears one or more chiral groups and is chosen so that its configuration is appropriate for the synthesis of a β-lactam with the desired absolute configuration. The absolute configuration thereof can be established by X-ray structure determination. Typically the (R,R) enantiomer of the chiral diimine leads to the (3R,4S) enantiomer of the corresponding β-lactam and vice versa.

By a "suitable" chiral β-lactam compound of formula Ia and Ib or Ic and Id, respectively, is meant a compound with the substituents $R_1$, $R_2$, $R_3$ and $R_4$ as is desired for the compounds of formula VIa and VIb or VIC and VId, respectively, or VIIa and VIIb, respectively.

By an enantioselective process is meant a process yielding a chiral compound.

The enantiomeric form of the trans- or cis-β-lactams produced in accordance with the invention refers to the configuration of the groups linked to the C-3 carbon atom and to the C-4 carbon atom of the trans- or cis-β-lactam obtained. The synthesis of a pure enantiomer of a β-lactam is important because of the potential for such a compound to be an antibiotic with a more specific activity.

Optionally substituted as used herein means unsubstituted or substituted by any group generally present in organic compounds, such as halogen, hydroxy, alkyl, aryl, aralkyl, alkoxy, aralkoxy and

heterocyclic groups.

As used herein, a heterocyclic group is a 5- or 6-membered heterocycle containing one or more heteroatoms, typically selected from O, N and S. The heterocycle can be fused to a second ring which may be heterocyclic or carbocyclic. Examples of heterocyclic groups within the present invention include thienyl, furyl, pyridyl, pyrryl, imidazolyl, pyridizinyl, pyrimidyl and pyrazinyl, all optionally substituted as defined above.

The anionic group in formula II above may be any anionic group which can act as a complexing ligand with the metal M, typically aluminum, zirconium, boron, tin or titanium, or which can form a salt with an alkali metal. Examples of such anionic groups include halide, thiolate, substituted sulphonate such as triflate, and alkoxide.

As used herein, the carbamate group is of the formula

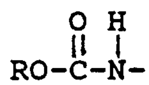

and the sulfonyl group of the formula

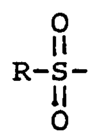

with R is (1-8C)alkyl, (6-18C)aryl or (1-8C)alkyl(1-8C)aryl, each optionally substituted.

It is very important that the metal compounds of formula $MW(X)_a(Y)_b(Z)_c$ used in the processes of the invention are substantially free of water. When small amounts of water are present, the product may be obtained in lower yield and the stereoselectivity of the process decreases. Preferably there is no more than 0.1% by weight of water present.

The reactions described above with the diimines to form 3-amino-4-imino-β-lactams are interesting because the 4-imino group represents a protected aldehyde function, which is suitable for further reaction.

It was surprising that by introducing a chiral group in $R_1$ or $R_4$ (see formula I) except for a very high diastereo-selectivity a large enantiomeric excess of more than 90% may be achieved. With the synthesis using a chiral imine or chiral α-diimine three important improvements in the synthesis of β-lactams have been achieved:

1. the formation of the trans-β-lactams (also obtained with the process described in EP-A-281177);
2. the formation of 3S,4R-trans- or cis-β-lactams and 3R,4S-trans- or cis-β-lactams;
3. the presence of easily convertible groups on position 4, formed after the hydrolysis of the imine group

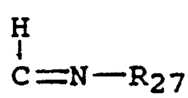

or the oxidation of the (1,2,O-isopropylidene)ethyl group, for instance.

The following Examples further illustrate the invention.

## Example I

Synthesis of trans-1-(substituted)-3-diethylamino-4-(substituted)-2-azetidinone (one-pot process)

13.33 ml (1.5 molar) of n-butyllithium in hexane, equivalent to 20 mmoles n-butyllithium was added to a solution of 2.02 g (20 mmoles) of diisopropylamine in 25 ml benzene. The resulting solution of lithium diisopropylamide so obtained was stirred for 10 minutes. Then 3.18 g (20 mmoles) of N,N-diethylglycine

ethyl ester was added giving an immediate white suspension. This suspension was stirred for 30 minutes. Then a solution of 2.73 g (20 mmoles) of zinc dichloride in 20 ml diethyl ether was added resulting in a yellow solution and this solution was stirred for another 10 minutes. Most of the diethyl ether and hexane present in solution was evaporated *in vacuo*. 3.26 g (20 mmoles) of N,N′-bis-t-butyl-1,4-diaza-1,3-butadiene was added to the resulting benzene solution and the reaction mixture refluxed for 30 minutes. During this period some solid material precipitated. Then most of the benzene was evaporated *in vacuo*. To the resulting suspension was added 30 ml of tetrahydrofuran to give a clear yellow solution, which was refluxed for another 30 minutes and then cooled down to room temperature and diluted with 20 ml of diethyl ether. 15 ml of a saturated aqueous ammonium chloride solution was added to the reaction mixture. The organic phase was separated and was washed twice with 15 ml portions of a saturated aqueous ammonium chloride solution and twice with 15 ml portions of water, dried with sodium sulphate and concentrated *in vacuo* yielding 5.20 g (92%) of the trans-2-azetidinone product as a white solid.

Following the same procedure and using the same reaction conditions as used above, one more 1,4-substituted *trans*-β-lactam was obtained, see Table I.

Table I

| | | yield | cis | trans |
|---|---|---|---|---|
| 1. | $R_1$ = t-butyl, $R_4$ = t-butylimino | 92% | 8% | 92% |
| 2. | $R_1$ = t-butyl, $R_4$ = 2-pyridyl | 80% | 0% | 100% |

Addendum to Table I: physical data of compounds 1 and 2.

trans-1-t-butyl-3-diethylamino-4-t-butylimino-2-azetidinone:

$^1$H NMR ($CDCl_3$): δ 7.47 (db, J = 7.6 Hz, 1H, H-C=N-t-Bu), 4.19 (dd, J = 7.6 and 1.8 Hz, 1H, N-CH-CH-CH=N), 3.86 (d, J = 1.8 Hz, 1H, N-CH-CH-CH=N), 2.65 (q, 4H, N-$CH_2$-$CH_3$), 1.26 (s, 9H, N-t-Bu), 1.51 (s, 9H, N-t-Bu), 0.97 (t, 6H, N-$CH_2$-$CH_3$).

$^{13}$C NMR ($CDCl_3$): δ 167.74 (C=O); 158.31 (C=N); 73.09 (C-C=N); 58.14 (O=C-C); 57.04 and 54.27 (C-($CH_3$)$_3$); 43.46 ($NCH_2$); 29.12 and 28.38 (C($CH_3$)$_3$); 12.13 (N-$CH_2$-$CH_3$).

trans-1-t-butyl-3-diethylamino-4-(2-pyridyl)-2-azetidinone:

m.p.: 51 °C. $^1$H NMR ($CDCl_3$): δ 8.54 (db, 1H), 7.73-7.64 and 7.34-7.16 (mp, 1H, mp, 2H, protons of the pyridyl group), 4.71 (db, 1H, J = 1.6 Hz, O=C-CH), 3.97 (db, 1H, J = 1.6 Hz, -CH-pyridyl), 2.79-2.64 (dqt, 4H, J = 7.1 Hz, $NCH_2$-$CH_3$), 1.22 (s, 9H, C($CH_3$)$_3$), 0.96 (tp, 6H, J = 7.1 Hz, N-$CH_2$-$CH_3$).

$^{13}$C NMR ($CDCl_3$): δ 169.24 (C=O), 160.34 ($C_2$ of the pyridyl group), 149.52, 136.79, 122.66, 120.84 (carbons of the pyridyl group), 78.21 (O=C-CH), 59.18 (-CH-pyridyl), 54.60 (C($CH_3$)$_3$), 43.74 ($NCH_2$), 28.20 (C($CH_3$)$_3$), 12.30 (N-$CH_2$-$CH_3$).

## Example II

### Synthesis of trans-3-diethylamino-4-substituted-2-azetidinone (one-pot process)

Following the same procedure and using the same reaction conditions as used in Example I, 1-unsubstituted *trans*-β- lactams were obtained by carrying out the reaction with N-trimethylsilyl protected imines. The N-$SiMe_3$ group is hydrolysed during the aqueous work up. Representative examples are given in Table II.

Table II

| | | yield | cis | trans |
|---|---|---|---|---|
| 1 | $R_4$ = 2-thienyl | 98% | 6% | 94% |
| 2 | $R_4$ = 2-furyl | 92% | 20% | 80% |

Addendum to Table II: physical data of compounds 1 and 2.

trans-3-diethylamino-4-(2-thienyl)-2-azetidinone:

m.p.: 101 °C. $^1$H NMR ($CDCl_3$): δ 7.26-7.22 and 7.01-6.98 (mp, 1H, mp, 2H, protons of the thienyl group), 6.77 (br.s, 1H, NH), 4.91 (db, 1H, J = 1.85 Hz, O=C-CH-), 4.13 (db, 1H, J = 1.85 Hz, O=C-C-CH-), 2.75 (qt, 4H, J = 7.1 Hz, N-$CH_2$-$CH_3$), 1.03 (tp, 6H, J = 7.1 Hz, N-$CH_2$-$CH_3$).

$^{13}$C NMR ($CDCl_3$): δ 169.81 (C=O), 143.89 ($C_2$ of the thienyl group), 127.19, 124.86, 124.54 (carbon atoms of the thienyl group), 82.49 (Et-N-CH-), 52.02 (-CH-thienyl), 43.94 ($NCH_2$), 12.55 (N-$CH_2$-$CH_3$).

| Analysis : | | | | |
|---|---|---|---|---|
| found : | C 58.58 | H 7.24 | N 12.40 | S 13.97 |
| calculated: | 59.90 | 7.19 | 12.49 | 14.29 |

trans-3-diethylamino-4-(2-furyl)-2-azetidinone:

trans-isomer: m.p. 123 °C. $^1$H NMR ($CDCl_3$): δ 7.39-7.37 and 6.35-6.28 (mp, 1H, mp, 2H, protons of the furyl group), 6.50 (br.s, 1H, NH), 4.66 (db, 1H, J = 2.0 Hz, O=C-CH-), 4.32 (mp, 1H, N-CH-furyl), 2.74 (2 x qt, 4H, J = 7.2 Hz, N-$CH_2$-$CH_3$), 1.03 (tp, 6H, J = 7.1 Hz, N-$CH_2$-$CH_3$).

$^{13}$C NMR ($CDCl_3$): δ 169.80 (C=O), 152.25 ($C_2$ of the furyl group), 142.67, 110.55, 107.44 (carbon atoms of the furyl group), 78.92 (N-CH-C=O), 49.29 (-CH-furyl), 43.63 (N-$CH_2$-$CH_3$), 12.35 (N-$CH_2$-$CH_3$).

| Analysis : | | | |
|---|---|---|---|
| found : | C 63.41 | H 7.82 | N 13.38 |
| calculated: | 63.44 | 7.74 | 13.45 |

## Example III

### Synthesis of trans-1-t-butyl-3-(2,2,5,5-tetramethyl-1-aza-2,5-disilacyclopentyl)-4-N-t-butylimino-2-azetidinone

10 mmoles of n-butyllithium (6.67 ml of a 1.5 molar solution in hexane) was added to a stirred solution containing diisopropylamine (1.40 ml; 10 mmoles) and 25 ml of diethyl ether at -70 °C. This reaction mixture was stirred for 10 minutes and then 2,2,5,5-tetramethyl-1-aza-2,5-disilacyclopentane-1-acetic acid ethyl ester (2.45 g; 10 mmoles) was added. The solution was stirred for another 15 minutes at -70 °C and then 10 mmoles of zinc dichloride (6.67 ml of a 1.48 molar solution in diethyl ether) was added. After 5 minutes at -70 °C, a white solid (LiCl) began to precipitate. Then bis-t-butyl-1,4-diaza-1,3-butadiene (1.68 g; 10 mmoles) was added. The reaction mixture was stirred for another 15 minutes at -70 °C and, after being warmed up to room temperature, quenched with 20 ml of a saturated aqueous ammonium chloride solution. The water layer was extracted with diethyl ether. The diethyl ether extract was washed with water, dried with sodium sulphate and concentrated *in vacuo* to give 3.45 g (94%) of the pure 2-azetidinone product as a pale yellow solid.

Following the same procedure and using the same reaction conditions as used above and for the 1-unsubstituted trans-β-lactam as described in Example II, two more 3-(2,2,5,5-tetra methyl-1-aza-2,5-disilacyclopentyl) trans-β-lactams were obtained, see Table III.

Table III

| | yield | cis | trans |
|---|---|---|---|
| $R_1$ = t-butyl $R_4$ = (t-butyl)imino | 94% | <2% | >98% |
| $R_1$ = t-butyl $R_4$ = 2-pyridyl | 100% | <2% | >98% |
| $R_1$ = -- $R_4$ = 2-pyridyl | 96% | <2% | >98% |

trans-1-(t-butyl)-3-(2,2,5,5-tetramethyl-1-aza-2,5-disilacyclopentyl)-4-(t-butyl)imino-2-azetidinone:

m.p. 99 °C. $^1$H NMR ($CDCl_3$): δ 7.46 (db, 1H, J = 7.8 Hz, HC=N), 4.00 (db, 1H, J = 1.8 Hz, O=C-CH), 3.82 (ddb, 1H, J = 7.8 and 1.8 Hz, -CH-C=N), 1.31 (s, 9H, $C(CH_3)_3$), 1.21 (s, 9H, $C(CH_3)_3$), 0.78 - 0.61 (mp, 4H, 2 x $SiCH_2$), 0.12 (s, 6H, 2 x $SiCH_3$), 0.09 (s, 6H, 2 x $SiCH_3$).

$^{13}$C NMR ($CDCl_3$): δ 168.57 (C=O), 158.12 (C=N), 66.62 (O=C-CH), 64.44 (CH-C=N), 57.35 ($C(CH_3)_3$), 54.13 ($C(CH_3)_3$), 29.34 ($C(CH_3)_3$), 28.62 ($C(CH_3)_3$), 7.98 ($SiCH_2$), 0.81 ($SiCH_3$), 0.15 ($SiCH_3$).

| Analysis : | | | | |
|---|---|---|---|---|
| found :<br>calculated: | C 57.43<br>58.80 | H 10.29<br>10.14 | N 11.47<br>11.43 | Si 15.32<br>15.28 |

trans-1-(t-butyl)-3-(2,2,5,5-tetramethyl-1-aza-2,5-disilacyclopentyl)-4-(2-pyridyl)-2-azetidinone:

m.p. 109 °C. $^1$H NMR ($CDCl_3$): δ 8.59-8.56, 7.73-7.64 and 7.32-7.17 (mp, 1H, mp, 1H, mp, 2H, protons of the pyridyl group), 4.30 (db, 1H, J = 1.85 Hz, O=C-CH-), 4.13 (mp, 1H, -CH-pyridyl), 1.21 (s, 9H, $C(CH_3)_3$), 0.78-0.61 (mp, 4H, $SiCH_2$), 0.09, 0.03 (2 x s, $SiCH_3$).

$^{13}$C NMR ($CDCl_3$): δ 169.94 (C=O), 159.68 ($C_2$ of the pyridyl group), 149.56, 136.57, 122.80, 121.25 (carbon atoms of the pyridyl group), 69.61 (O=C-CH-), 67.18 (-CH-pyridyl), 54.24 ($C(CH_3)_3$), 28.27 ($C(CH_3)_3$), 8.02 ($SiCH_2$), 0.60 ($SiCH_3$), 0.29 ($SiCH_3$).

trans-3-(2,2,5,5-tetramethyl-1-aza-2,5-disilacyclopentyl)-4-(2-pyridyl)-2-azetidinone:

m.p. 102 °C. $^1$H NMR ($CDCl_3$): δ 8.57-8.55, 7.69-7.64 and 7.31-7.18 (mp, 1H, mp, 1H and mp, 2H, protons of the pyridyl group), 6.81 (br, s, 1H, NH), 4.46 (db, 1H, J = 2 Hz, O=C-CH-), 4.24 (mp, 1H, -CH-pyridyl), 0.78-0.68 (mp, 4H, $SiCH_2$), 0.09 (s, 6H, $SiCH_3$), 0.03 (s, 6H, $SiCH_3$).

$^{13}$C NMR ($CDCl_3$): δ 171.62 (C=O), 158.56 ($C_2$ of the pyridyl group), 149.60, 136.84, 122.96 and 120.44 (carbon atoms of the pyridyl group), 72.37 (O=C-CH-), 63.58 (-CH-pyridyl), 7.95 ($SiCH_2$), 0.43, 0.32 ($SiCH_3$).

## Example IV

Synthesis of trans-(3R,4S)-1-(α-(R)methylbenzyl)-3-(2,2,5,5-tetramethyl-1-aza-2,5-disilacyclopentyl)-4-N-(α-(R)methylbenzyl)imino-2-azetidinone

In the same way as described in Example III, trans-(3R,4S)-1-(α-(R)methylbenzyl)-3-(2,2,5,5-tetramethyl-1-aza-2,5-disilacyclopentyl)-4-N-(α-(R)methylbenzyl)imino-2-azetidinone was prepared, using N,N′-bis(R,R′)-(α-methylbenzyl)-1,4-diaza-1,3-butadiene, with a yield of 90%, comprising the 3R,4S component in an excess of 86%.

Regarding the optical purity of the starting α-methylbenzyldiazabutadiene of 93%, the optical yield of the 3R,4S component is more than 90%.

$^1$H NMR ($CDCl_3$): δ 7.53 (d, 1H, J = 7.6 Hz, HC=N), 7.41-7.13 (m, 10H, arom.), 4.85 (q, 1H, J = 7.2 Hz, HC(Me)Ph), 4.34 (q, 1H, J = 6.6 Hz, HC(Me)Ph), 4.18 (d, 1H, J = 1.9 Hz, $C^3$H), 3.67 (dd, 1H, J = 7.6 and 1.9 Hz, $C^4$H), 1.50 (d, 3H, $CH_3C(H)Ph$), 1.39 (d, 3H, $CH_3C(H)Ph$), 0.76-0.52 (m, 4H, $SiCH_2CH_2Si$), 0.05 (s, 6H, $Si(CH_3)_2$), -0.05 (s, 6H, $Si(CH_3)_2$). $^{13}$C NMR ($CDCl_3$): δ 168.74 (C=O), 161.10 (C=N), 143.81, 139.84, 128.70, 128.54, 127.87, 127.37, 127.22, 126.63, (carbons of both phenyl groups), 69.82 and 66.16 (HC(Me)-Ph 2x), 65.56 ($C^3$), 52.47 ($C^4$), 24.18 and 19.88 (HC(Me)Ph 2x) 7.94 ($SiCH_2CH_2Si$), 0.66 and -0.09 ($Si(CH_3)_2$

2x).
$\alpha_D^{20}$ = +4.17° (C = 10, ethanol).

## Example V

### Synthesis of trans-(3R,4S)-1-(α-(R)methylbenzyl)-3-(2,2,5,5-tetramethyl-1-aza-2,5-disilacyclopentyl)-4-(2-pyridyl)-2-azetidinone

In the same way as described in Example III, trans-(3R,4S)-1-(α-(R)methylbenzyl)-3-(2,2,5,5-tetramethyl-1-aza-2,5-disilacyclopentyl)-4-(2-pyridyl)-2-azetidinone was prepared, using N-(α-(R)methylbenzyl)-1,4-diaza-1,3-butadiene, with a yield of 98%, comprising the 3R,4S component in an excess of much more than 95%. m.p.: 132°C. $^1$H NMR ($CDCl_3$): δ 8.62-8.59 (mp, 1H), 7.67-7.58 (mp, 1H), 7.32-7.09 (mp, 7H protons pyridyl and phenyl group), 5.01 (q, 1H, J = 7.2 Hz, H̲C-$CH_3$), 4.36 (db, 1H, J = 2.0 Hz, O=C-CH̲), 4.04 (db, 1H, J = 2.0 Hz, C-CH̲-pyridyl), 1.26 (db, 3H, J = 7.2 Hz, HC-$CH_3$), 0.70-0.56 (mp, 4H, $SiCH_2CH_2Si$), -0.09 and -0.13 (2 x s, 12H, $Si(CH_3)_2$).
$^{13}$C NMR ($CDCl_3$): δ 170.14 (C=O), 158.24, 149.74, 139.76, 136.42, 128.56, 127.78, 127.52, 122.97, 121.94 (carbons of pyridyl and phenyl group), 69.89 (O=C-C̲H), 66.90 (C̲H-pyridyl), 52.27 (HC̲-Me), 18.79 (HC-M̲e̲), 7.93 ($SiCH_2CH_2Si$), 0.43 and -0.04 ($Si(CH_3)_2$ 2x).
$\alpha_D^{20}$ = +53.36° (C = 10, ethanol).

The absolute configuration of trans-(3R,4S)-1-(α-(R)-methylbenzyl)-3-(2,2,5,5-tetramethyl-1-aza-2,5-disilacyclopentyl)-4-(2-pyridyl)-2-azetidinone and therefore also of trans-(3R,4S)-1-(α-(R)methylbenzyl)-3-(2,2,5,5-tetramethyl-1-aza-2,5-disilacyclopentyl)-4-N-(α-(R)methylbenzyl)imino-2-azetidinone were determined to be 3R,4S from an X-ray structure determination*, the result of which is shown in the Figures IA and IB.

* Crystal data, collection and refinement for this compound: $C_{22}H_{31}N_3OSi_2$, M = 409.68, monoclinic, space group I2, a = 18.410(1), b = 6.813(1), c = 19.471(1) Å, $\beta$ = 105.77(1)°, U = 2350.3(4) Å$^3$, Z = 4, F(000) = 880, $D_c$ = 1.158 g cm$^{-3}$, T = 295 K, Mo-$K_\alpha$ (Zr-filtered) radiation (wavelength = 0.71073Å), $\mu$(Mo-$K_\alpha$) = 1.40 cm$^{-1}$.
Enraf-Nonius CAD4F diffractometer in the range 2.7≤2Θ≤55°. 2295 unique reflections ($R_{av}$ = 0.04) with I ≥ 2.5σ (I) were used in structure solution (direct methods; SHELXS-86) and weighted full-matrix least-squares refinement (SHELX-76), which converged at R and $R_w$ values of 0.046 and 0.044, respectively. The enantiomorph was chosen on the basis of the configuration of the starting (R)α-methylbenzylamine.

## Example VI

### Synthesis of trans-(3R,4S)-1-p-methoxyphenyl-3-(2,2,5,5-tetramethyl-1-aza-2,5-disilacyclopentyl)-4-[(1S)-(1,2,O-isopropylidene)ethyl]-2-azetidinone

10 mmoles of n-butyllithium (6.67 ml of a 1.5 molar solution in hexane) was added to a stirred solution containing diisopropylamine (1.40 ml; 10 mmoles) and 40 ml of diethyl ether at -78°C. This reaction mixture was stirred for 5 minutes and then 2,2,5,5-tetramethyl-1-aza-2,5-disilacyclopentane-1-acetic acid ethyl ester (2.45 g; 10 mmoles) was added. The solution was stirred for another 15 minutes at -78°C and then 10 mmoles of zinc dichloride (6.10 ml of a 1.64 molar solution in diethyl ether) was added. After 45 minutes at -78°C a white solid (LiCl) began to precipitate. Then 10 mmoles of N[(2S)-(2,3-O-isopropylidene)propylidene]-p-methoxyphenylamine (20.0 ml of a 0.5 molar solution in diethyl ether) was slowly added (30 minutes). The reaction mixture was stirred for 1 hour at -78°C and, after being warmed up to room temperature, quenched with 25 ml of a saturated aqueous ammonium chloride solution. The water layer was extracted with diethyl ether/benzene (1/1 vv). The organic extract was dried with sodium sulphate and concentrate *in vacuo* to yield 4.18 g (96%) of the 2-azetidinone product as a pale brown solid.

The $^1$H NMR spectrum revealed that the product was a mixture of three diastereomer compounds: cis (3S,4S), cis (3R,4R) and trans (3R,4S) in a ratio of 3.5:3.5:93.

The enantiomerically pure trans-product was obtained as white crystals after recrystallization from

diethyl ether in 85% yield.

m.p. 164° C. $^1$H NMR ($CDCl_3$): δ 7.36 (d, 2H, J = 9.0 Hz, ArH), 6.86 (d, 2H, J = 9.0 Hz, ArH), 4.52 (ddd, 1H, J = 3.2, 6.8 and 6.9 Hz, -C(O)H-$CH_aH_bO$), 4.31 (d, 1H, J = 2.3 Hz, N-CH-CH-R*), 4.06 (dd, 1H, J = 6.8 and 8.2 Hz, -C(O)H-$CH_aH_bO$), 3.99 (dd, 1H, J = 2.3 and 3.2 Hz, N-CH-CH-R*), 3.78 (s, 3H, $OCH_3$), 3.75 (dd, J = 6.9 and 8.2 Hz, -C(O)H-$CH_aH_bO$), 1.37 and 1.31 ($C(CH_3)_2$), 0.76 (s, 4H, $SiCH_2CH_2Si$), 0.16 and 0.13 ($Si(CH_3)_2$.

$^{13}$C NMR ($CDCl_3$): δ 167.74 (C=O), 156.54, 130.52, 119.88, 114.40 (ArC), 109.81 ($CMe_2$), 73.45 (-C(O)H-$CH_2O$), 65.68 (N-CH-CH-R*), 63.00 (N-CH-CH-R*), 62.39 (-C(O)H-$CH_2O$), 55.45 ($OCH_3$), 26.08 and 24.81 (C-$(CH_3)_2$), 7.98 ($SiCH_2CH_2Si$), 0.93 and 0.03 ($Si(CH_3)_2$).

$[\alpha_D]^{20}$ = -9.21° (C = 0.9, benzene).

## Example VII

### A. Synthesis of trans-(3R,4R)-1-((R)-α-methylbenzyl)-3-(2,2,5,5-tetramethyl-1-aza-2,5-disilacyclopentyl)-4-methyl-2-azetidinone

Following the same procedure as described in Example III, trans-1-(3R,4R)-1-((R)-α-methylbenzyl)-3-(2,2,5,5-tetramethyl-1-aza-2,5-disilacyclopentyl)-4-methyl-2-azetidinone was prepared in diethyl ether as solvent, using chiral N-(ethylidene)-(R)-α-methylbenzylamine as imine reagent. The 2-azetidinone product was isolated as a brown oil with a yield of 90%.

The $^1$H NMR spectrum revealed that the product was a mixture of three diastereomeric products; one single trans-isomer (80%, > 95% enantiomeric excess) and two cis-isomers (20%, = 0% enantiomeric excess).

$^1$H NMR ($CDCl_3$): trans, δ 7.40-7.10 (m, 5H, ArH), 4.86 (q, 1H, J = 7.2 Hz, HC(Me)Ph), 3.69 (d, 1H, J = 2.0 Hz, N-CH-CH-Me), 3.15 (dq, 1H, J = 6.3 and 1.9 Hz, N-CH-CH-Me), 1.63 (d, 3H, J = 7.2 Hz, HC(Me)Ph), 1.24 (d, 3H, J = 6.3 Hz, N-CH-CH-Me), 0.76-0.52 (m, 4H, $SiCH_2CH_2Si$), 0.05 and -0.05 (s, 6H, $Si(CH_3)_2$).

### B. Synthesis of trans-(3R,4R)-1-((R)-α-methylbenzyl)-3-amino-4-methyl-2-azetidinone

Trans-1-(3R,4R)-1-((R)-α-methylbenzyl)-3-(2,2,5,5-tetramethyl-1-aza-2,5-disilacyclopentyl)-4-methyl-2-azetidinone (3.05 g (crude product); 8.8 mmoles) was dissolved in 25 ml of tetrahydrofuran. After addition of hydrochloric acid (5 ml of an 4.0 molar solution in water) the mixture was stirred for one hour at room temperature. After addition of 25 ml of diethyl ether, the water layer was separated, washed with 25 ml of diethyl ether and then made basic with ammonia (25% in water). The water layer was extracted with dichloro methane. The dichloromethane extract was dried with magnesiumsulfate and concentrated *in vacuo* to afford 1.46 g (81%) of the 2-azetidinone product as a dark brown oil.

The $^1$H NMR spectrum revealed that the product was a mixture of three diastereomeric products; one single trans-isomer (80%, > 95% enantiomeric excess) and two cis-isomers (20%, ≈ 0% enantiomeric excess).

$^1$H NMR ($CDCl_3$): trans, δ 7.40-7.10 (m, 5H, ArH), 4.86 (q, 1H, J = 7.2 Hz, HC(Me)Ph), 3.53 (d, 1H, J = 2.0 Hz, N-CH-CH-Me), 3.13 (dq, 1H, J = 6.2 and 2.0 Hz, N-CH-CH-Me), 1.58 (d, 3H, J = 7.2 Hz, HC(Me)Ph), 1.50 (br.s, 2H, $NH_2$), 1.22 (d, 3H, J = 6.2 Hz, N-CH-CH-Me).

### C. Synthesis of cis-(3R,4S)-1-((R)-α-methylbenzyl)-3-(2,2,5,5-tetramethyl-1-aza-2,5-disilacyclopentyl)-4-methyl-2-azetidinone

Following the same procedure as described in Example III, cis-1-(3R,4S)-1-((R)-α-methylbenzyl)-3-(2,2,5,5-tetramethyl-1-aza-2,5-disilacyclopentyl)-4-methyl-2-azetidinone was prepared in tetrahydrofuran as solvent, using chiral N-(ethylidene)-(R)-α-methylbenzylamine as imine reagent. The 2-azetidinone product was isolated as a brown oil with a yield of 95%.

The $^1$H NMR spectrum revealed that the product was a mixture of three diastereomeric products; one single cis-isomer (85%, > 95% enantiomeric excess) and two trans-isomers (15%, 75% enantiomeric excess).

$^1H$ NMR ($CDCl_3$): cis, δ 7.40-7.10 (m, 5H, ArH), 4.62 (q, 1H, J = 7.3 Hz, HC(Me)Ph), 4.35 (d, 1H, J = 4.9 Hz, N-CH-CH-Me), 3.50 (dq, 1H, J = 6.3 and 4.9 Hz, N-CH-CH-Me), 1.69 (d, 3H, J = 7.3 Hz, HC(Me)Ph), 0.88 (d, 3H, J = 6.3 Hz, N-CH-CH-Me), 0.76-0.52 (m, 4H, $SiCH_2CH_2Si$), 0.05 and -0.05 (s, 6H, $Si(CH_3)_2$).

D. Synthesis of cis-1-(3R,4S)-1-((R)-α-methylbenzyl)-3-amino-4-methyl-2-azetidinone

Following the same procedure as described for the trans-compound cis-1-(3R,4S)-1-((R)-α-methylbenzyl)-3-amino-4-methyl-2-azetidinone was prepared from cis-1-((3R,4R)-α-methylbenzyl)-3-(2,2,5,5-tetramethyl-1-aza-2,5-disilacyclopentyl)-4-methyl-2-azetidinone. The 2-azetidinone product was isolated as a dark brown oil with a yield of 87%.

The $^1H$ NMR spectrum revealed that the product was a mixture of three diastereomeric products; one single cis-isomer (85%, > 95% enantiomeric excess) and two trans-isomers (15%, 75% enantiomeric excess).

$^1H$ NMR ($CDCl_3$): cis, δ 7.40-7.10 (m, 5H, ArH), 4.65 (q, 1H, J = 7.2 Hz, HC(Me)Ph), 4.09 (d, 1H, J = 5.0 Hz, N-CH-CH-Me), 3.66 (dq, 1H, J = 6.2 and 5.0 Hz, N-CH-CH-Me), 1.67 (d, 3H, J = 7.2 Hz, HC(Me)Ph), 1.50 (br.s, 2H, $NH_2$), 0.94 (d, 3H, J = 6.2 Hz, N-CH-CH-Me).

## Example VIII

A. Synthesis of trans-(3S,4S)-1-((S)-α-methylbenzyl)-3-(2,2,5,5-tetramethyl-1-aza-2,5-disilacyclopentyl)-4-ethyl-2-azetidinone.

According to the procedure of Example VII A, using N-(propylidene)-(S)-α-methylbenzylamine as imine reagent, the 2-azetidinone product was isolated in 92% yield (3.33 g) as a brown oil.

The $^1H$ NMR spectrum revealed that the product was a mixture of three diastereomeric products; one single trans-isomer (95%, > 95% enantiomeric excess and two cis-isomers (5%, ≈ 0% enantiomeric excess).

$^1H$ NMR ($CDCl_3$): trans, δ 7.40-7.10 (m, 5H, ArH), 4.77 (q, 1H, J = 7.2 Hz, HC(Me)Ph), 3.75 (d, 1H, J = 2.3 Hz, N-CH-CH-Et), 3.24 (ddd, 1H, J = 8.5, 3.8 and 2.3 Hz, N-CH-CH-Et), 1.61 (d, 3H, J = 7.2 Hz, HC(Me)-Ph), 1.80-1.20 (m, 2H, $CH_2CH_3$), 0.82 (t, 3H, J = 7.4 Hz, $CH_2CH_3$), 0.73-0.67 (m, 4H, $SiCH_2CH_2Si$), 0.02 and -0.03 (s, 6H, $Si(CH_3)_2$).

B. Synthesis of cis-(3S,4R)-1-((S)-α-methylbenzyl)-3-(2,2,5,5-tetramethyl-1-aza-2,5-disilacyclopentyl)-4-ethyl-2-azetidinone.

Following the procedures of Example VII using N-(propylidene)-(S)-α-methylbenzylamine as imine reagent, but with the addition of 5 ml of hexamethylphosphoric triamide (HMPA) before addition of 3-(2,2,5,5-tetramethyl)-1-aza-2,5-disilacyclopentane-1-acetic acid ethyl ester to the solution of LDA, the cis-2-azetidinone product was isolated exclusively in 95% yield (3.38 g) as a brown oil.

The $^1H$ NMR spectrum showed the presence of the one single diastereomer (e.e. > 95%).

$^1H$ NMR ($CDCl_3$): cis, δ 7.40-7.10 (m, 5H, ArH) , 4.60 (q, 1H, J = 7.2 Hz, HC(Me)Ph), 4.28 (d, 1H, J = 4.9 Hz, N-CH-CH-Et), 3.21 (ddd, 1H, J = 8.9, 4.9 and 4.0 Hz, N-CH-CH-Et), 1.68 (d, 3H, J = 7.2 Hz, HC(Me)-Ph), 1.80-1.30 (m, 2H, $CH_2CH_3$), 0.76 (t, 3H, J = 7.5 Hz, $CH_2CH_3$), 0.75-0.60 (m, 4H, $SiCH_2CH_2Si$), 0.07 and 0.03 (s, 6H, $Si(CH_3)_2$).

## Example IX

A. Synthesis of trans-(3R,4R)-1-((R)-α-methylbenzyl)-3-methylcarbamate-4-methyl-2-azetidinone

Trans-1-(3R,4R)-1-((R)-α-methylbenzyl)-3-amino-4-methyl-2-azetidinone (1.68 g (crude product); 8.24

mmoles) was dissolved in 25 ml of benzene. After addition of methylchloro formate (0.7 ml, 9.06 mmoles) ans excess of triethylamine (2.5 ml, 18 mmoles) is slowly added at room temperature. Immediately a white solid ($Et_3N.HCl$) starts to precipitate. After stirring for one hour at room temperature all volatile material was removed in vacuo and the solid residue was extracted with diethyl ether. Isolated yield 95%. The pure trans-product is obtained as a white crystalline compound after addition of a small amount of n-pentane to the diethyl ether extract and cooling to -30° C.

m.p. 135° C. $^1H$ NMR ($CDCl_3$): trans, δ 7.34-7.24 (m, 5H, ArH), 5.81 (d, 1H, J = 6.4 Hz, NH), 4.88 (q, 1H, J = 7.2 Hz, HC(Me)Ph), 4.20 (dd, 1H, J = 6.4 and 1.9 Hz, N-CH-CH-Me), 3.62 (s, 3H, OMe), 3.39 (dq, J = 6.2 and 1.9 Hz, N-CH-CH-Me), 1.61 (d, 3H, J = 7.2 Hz, HC(Me)Ph), 1.26 (d, 3H, J = 6.2 Hz, N-CH-CH-Me).

$^{13}C$ NMR ($CDCl_3$): δ 165.79 (C=O), 156.58 (COOMe), 139.69, 128.73, 127.76, 127.04 (ArC), 63.37 (N-CH-CH-Me), 57,82 (N-CH-CH-Me), 52.39 (C(H)(Me)Ph), 51.85 (OMe), 91.51 (C(H)(Me)Ph), 18.94 (N-CH-CH-Me).

[$^{\alpha}D]^{25}$ = +51.2 (c = 1.0 in benzene).

| Analysis : | | | | |
|---|---|---|---|---|
| found : | C 63.55 | H 6.81 | N 10.61 | O 18.77 |
| calculated: | 64.11 | 6.92 | 10.68 | 18.30 |

B. Synthesis of cis-(3R,4S)-1-((R)-α-methylbenzyl)-3-methylcarbamate-4-methyl-2-azetidinone

Following the same procedure as described in Example VII C cis-(3R,4S)-1-((R)-α-methylbenzyl)-3-methylcarbamate-4-methyl-2-azetidinone was prepared with a yield of 95%. The pure cis-product is obtained as a white crystalline compound after addition of a small amount of n-pentane to the diethyl ether extract and cooling to -30° C.

m.p. 100° C. $^1H$ NMR ($CDCl_3$): cis, δ 7.31-7.24 (m, 5H, ArH), 5.84 (d, 1H, J = 8.0 Hz, NH), 4.92 (dd, 1H, J = 8.0 and 4.9 Hz, N-CH-CH-Me), 4.65 (q, 1H, J = 7.1 Hz, HC(Me)Ph), 3.76 (dq, 1H, J = 5.7 and 4.9 Hz, N-CH-CH-Me), 3.64 (s, 3H, OMe), 1.67 (d, 3H, J = 7.1 Hz, HC(Me)Ph), 0.93 (d, 3H, J = 6.3 Hz, N-CH-CH-Me).

$^{13}C$ NMR ($CDCl_3$): δ 166.43 (C=O), 156.83 (COOMe), 140.70, 128.76, 127.82, 126.76 (ArC), 59.24 (N-CH-CH-Me), 53.07 (N-CH-CH-Me), 52.51 (C(H)(Me)Ph) and (OMe), 19.08 (C(H)(Me)Ph), 14.10 (N-CH-CH-Me).

[$^{\alpha}D]^{25}$ = -6.1 (c = 1.0 in chloroform).

C. Synthesis of trans-(3R,4R)-3-methylcarbamate-4-methyl-2-azetidinone

Trans-1-((R)-α-methylbenzyl)-3-methylcarbamate-4-methyl-2-azetidinone (0.67 g (crude product); 2.56 mmoles) was dissolved in 10 ml of tetrahydrofuran. After addition of about 20 ml of liquid ammonia, small pieces of sodium metal are added until the blue color persists. The ammonia was evaporated and the residue quenched with 20 ml of a saturated aqueous ammonium chloride solution. After addition of 20 ml of dichloromethane, the organic layer was separated and the water layer was extracted with two 20 ml portions of dichloromethane. The combined extracts were dried with sodium sulphate and concentrated in vacuo yielding 0.36 g (90%) of the 2-azetidinone product. The product was purified by recrystallization from chloroform/pentane.

$^1H$ NMR (acetone-$d_6$); trans, δ 7.22 (br.s, 1H, NH), 6.96 (br.d, 1H, NH), 4.25 (dd, 1H, J = 8.6 and 2.2 Hz, N-CH-CH-Me), 3.66 (dq, 1H, J = 6.2 and 2.2 Hz, N-CH-CH-Me), 3.61 (s, 3H, OMe), 1.35 (d, 3H, J = 6.2 Hz, N-CH-CH-Me).

$^{13}C$ NMR (acetone-$d_6$): δ 167.09 (C=O), 157.11 (COOMe), 66.03 (N-CH-CH-Me), 53.35 (N-CH-CH-Me), 52.22 (OMe), 19.63 (N-CH-CH-Me).

D. Synthesis of cis-(3R,4S)-3-methylcarbamate-4-methyl-2-azetidinone

Following the same procedure described above cis-(3R,4S)-3-methylcarbamate-4-methyl-2-azetidinone was obtained in 93% yield as an off-white solid.

$^1H$ NMR (acetone-$d_6$): cis, δ 7.31 (br.s, 1H, NH), 7.02 (br.d, 1H, NH), 4.94 (dd, 1H, J = 9.5 and 5.3 Hz, N-CH-CH-Me), 3.89 (dq, 1H, J = 6.1 and 5.3 Hz, N-CH-CH-Me), 3.62 (s, 3H, OMe), 1.20 (d, 3H, J = 6.1 Hz, N-CH-CH-Me).

$^{13}C$ NMR (acetone-$d_6$): δ 167.73 (C=O), 157.45 (COOMe), 61.75 (N-CH-CH-Me), 52.33 (OMe), 50.30 (N-CH-CH-Me), 16.21 (N-CH-CH-Me).

## Example X

### A. Synthesis of trans-(3S,4S)-1-((S)-α-methylbenzyl)-3-amino-4-ethyl-2-azetidinone

Trans-1-((S)-α-methylbenzyl)-3-(2,2,5,5,-tetramethyl-1-aza-2,5-disilacyclopentyl)-4-ethyl-2-azetidinone (3.33 g (crude product); 9.2 mmoles) was dissolved in 25 ml of THF. After addition of hydrochloric acid (5 ml of an 4.0 molar solution in water) the mixture was stirred for one hour at room temperature. After addition of 25 ml of diethylether, the water layer was separated, washed with 25 ml of diethyl ether and then made basic with ammonia (25% in water). The water layer was extracted with dichloromethane. The dichloromethane extract was dried with magnesiumsulfate and concentrated in vacuo to afford 1.77 g (88%) of the 2-azetidinone product as a brown oil.

The $^1H$ NMR spectrum revealed that the product was a mixture of three diastereomeric products; one single trans-isomer (95%, >95% enantiomeric excess) and two cis-isomers (5%, ≈ 0% enantiomeric excess).

$^1H$ NMR ($CDCl_3$): trans, δ 7.40-7.10 (m, 5H, ArH), 4.85 (q, 1H, J = 7.2 Hz, HC(Me)Ph), 3.63 (d, 1H, J = 2.0 Hz, N-CH-CH-Et), 3.00 (ddd, 1H, J = 9.3, 3.8 and 2.0 Hz, N-CH-CH-Et), 1.59 (d, 3H, J = 7.2 Hz, HC(Me)-Ph), 1.80-1.20 (m, 4H, $CH_2CH_3$ and $NH_2$), 0.85 (t, 3H, J = 7.4 Hz, $CH_2CH_3$).

### B. Synthesis of trans-(3S,4S)-1-((S)-α-methylbenzyl)-3-methylcarbamate-4-ethyl-2-azetidinone

Trans-1-((S)-α-methylbenzyl)-3-amino-4-ethyl-2-azetidinone (1.77 g (crude product); 8.1 mmoles) was dissolved in 25 ml of benzene. After addition of methylchloroformate (0.7 ml, 9.1 mmoles) an excess of triethylamine (2.5 ml, 18 mmoles) is slowly added at room temperature. Immediately a white solid ($Et_3N.HC1$) starts to precipitate. After stirring for one hour at room temperature all volatile material was removed in vacuo and the solid residue was extracted with diethyl ether. Isolated yield 93%. The pure trans-product is obtained as a white crystalline compound after addition of a small amount of n-pentane to the diethylether extract and cooling to -30 °C.

$^1H$ NMR ($CDCl_3$): trans, δ 7.40-7.20 (m, 5H, ArH) , 5.29 (d, 1H, J = 7.1 Hz, NH), 4.87 (q, 1H, J = 7.2 Hz, HC(Me)Ph), 4.30 (dd, 1H, J = 7.4 and 2.1 Hz, N-CH-CH-Et), 3.65 (s, 3H, OMe), 3.25 (ddd, 1H, J = 9.2, 3.7 and 2.1 Hz, N-CH-CH-Et), 1.63 (d, 3H, J = 7.2 Hz, HC(Me)Ph), 1.8-1.2 (m, 2H, $CH_2CH_3$), 0.88 (t, 3H, J = 7.4 Hz, $CH_2CH_3$).

### C. Synthesis of trans-(3S,4S)-3-methylcarbamate-4-ethyl-2-azetidinone

Trans-1-((S)-α-methylbenzyl)-3-methylcarbamate-4-ethyl-2-azetidinone (2.06 g (crude product) was dissolved in 10 ml of tetrahydrofuran. After addition of about 20 ml of liquid ammonia, small pieces of sodium metal are added until the blue color persists. The ammonia was evaporated and the residue quenched with 20 ml of a saturated aqueous ammonium chloride solution. After addition of 20 ml of dichloromethane, the organic layer was separated and the water layer was extracted with two 20 ml portions of dichloromethane. The combined extracts were dried with sodium sulphate and concentrated in vacuo yielding 1.22 g (95%) of the 2-azetidinone product. The product was purified by recrystallization from chloroform/pentane.

$^1H$ NMR (acetone-$d_6$): trans, δ 7.36 (br.s, 1H, NH), 6.99 (br.d, 1H, NH), 4.32 (dd, 1H, J = 8.8 and 2.4 Hz, N-CH-CH-Et), 3.61 (s, 3H, OMe), 3.48 (dt, 1H, J = 6.7 and 2.4 Hz, N-CH-CH-Et), 1.67 (dq, 2H, J = 7.4 and 6.7 Hz, $CH_2CH_3$), 0.97 (t, 3H, J = 7.4 Hz, $CH_2CH_3$).

$^{13}C$ NMR (acetone-$d_6$): δ 167.44 (C=O), 157.04 (COOMe), 64.48 (N-CH-CH-Et), 59.19 (N-CH-CH-Et), 52.22 (OMe), 27.75 ($CH_2CH_3$), 10.46 ($CH_2CH_3$).

## Example XI

Synthesis of the 3-N-t-butylimino-3-N′-t-butylamino-2-N″,N″-diethylamino propionic acid ethyl ester zinc dichloride complex

To a stirred solution containing diisopropylamine (2.02 g; 20 mmoles) in 50 ml of dry benzene at room temperature was added 20 mmoles of n-butyllithium (13.33 ml of a 1.5 molar solution in hexane). After stirring for 10 minutes N,N-diethylglycine ethyl ester (3.18 g; 20 mmoles) was added. The resulting clear, pale yellow, solution was stirred for 15 minutes and then 20 mmoles of dry zinc dichloride (20.0 ml of a 1.0 molar solution in diethyl ether) was added, upon which a white solid (LiCl) began to precipitate. The suspension was stirred for another 15 minutes and then N,N′-ditert.butyl-1,4-diaza-1,3-butadiene (3.36 g; 20 mmoles) was added. The reaction mixture was stirred for 5 hours at 70 °C. After cooling to room temperature the reaction mixture was quenched with 40 ml of a saturated aqueous solution of ammonium chloride. The aqueous layer was separated and extracted with two 30 ml portions of benzene. The combined extracts were dried on sodium sulfate and concentrated in vacuo to afford a pale yellow oil. Upon addition of diethyl ether white crystals began to precipitate. The crystals were isolated on a glass-fritt, rinsed with diethyl ether and dried in vacuo. Yield 1.80 g (19%).

The $^1H$ NMR spectrum revealed that the product was a mixture of erythro/threo isomers (erythro/threo = 4/1). Recrystallization from hot benzene afforded the pure erythro isomer as colourless crystals.
m.p. 142 °C. $^1H$ NMR ($C_6D_6$): δ 7.96 (d, J = 2.3 Hz, 1H, HC=N), 4.41 (d, J = 9.6 Hz, 1H, EtOOCCH), 4.20 (br.d, J = 9.6 Hz, 1H, N-CH-C(H)=N), 4.06-3.86 (m, 2H, $OCH_2CH_3$), 2.97 (br.s, 1H, NH), 2.60-2.42 (m, 4H, $NCH_2CH_3$), 1.30 (s, 9H, t-Bu), 1.15 (s, 9H, t-Bu), 1.08-0.91 (m, 9H, $OCH_2CH_3$ and $NCH_2CH_3$).
$^{13}C$ NMR ($CDCl_3$): δ 170.75 (C=O), 168.72 (C=N), 65.96 (EtOOCCH), 61.55 ($OCH_2CH_3$), 60.64 ($C(CH_3)_3$), 56.12 ($C(CH_3)_3$), 55.46 (N-CH-C(H)=N), 45.77 ($NCH_2CH_3$), 29.27, 28.12 ($C(CH_3)_3$), 14.40 ($OCH_2CH_3$), 14.03 ($NCH_2CH_3$).

| Analysis : | | | | | |
|---|---|---|---|---|---|
| Found :<br>Calculated: | C 46.63<br>46.62 | H 7.82<br>7.82 | N 9.13<br>9.12 | Cl 15.14<br>15.14 | Zn 14.16<br>14.16 |

Following the same procedure as described above, however, now instead of N,N′-ditert.butyl-1,4-diaza-1,3-butadiene the zinc dichloride complex of N,N′-ditert.butyl-1,4-diaza-1,3-butadiene (6.08 g, 20 mmoles) was added, the 3-N-t-butylimino-3-N′-t-butylamino-2-N″,N″-diethylamino propionic acid ethyl ester zinc dichloride complex was isolated in 70% yield (6.65 g).

## Example XII

Conversion of trans-(3R,4S)-1-(α-(R)methylbenzyl)-amino-4-(2-pyridyl)-2-azetidinone into 2-(R)-amino-3-(2-pyridyl)propanoic acid (R)α-methylbenzylamide

To a stirred solution containing trans-(3R,4S)-1-(α-(R)-methylbenzyl)-3-amino-4-(2-pyridyl)-2-azetidinone (0.60 g; 2.2 mmoles) in 10 ml of dry methanol was added 0.60 g of Pd/C (10% paladium on carbon) and dry ammonium formiate (0.80 g; 12 mmoles). The reaction mixture was stirred for 3 hours at 70 °C. After cooling to room temperature the mixture was filtered over Celite, which was rinsed twice with 10 ml of diethyl ether. The organic layers were dried on sodium sulfate and concentrated in vacuo to afford 0.40 g (66%) of the crude product as colourless oil.
$^1H$ NMR ($CDCl_3$): δ 8.48 (d, 1H, pyrH), 7.79 (br.d, J = 8.0 Hz, 1H, O=CNH) 7.63-7.55 (m, 1H, pyrH), 7.33-7.10 (m, 7H, ArH and pyrH), 5.07 (dq, J = 6.9 and 8.0 Hz, 1H, CH(Me)(Ph)), 3.76 (dd, J = 8.2 and 4.2 Hz, 1H, O=C-CH-$NH_2$), 3.30 (dd, J = 4.2 and 14.0 Hz, 1H, $CH_aH_b$-pyr), 3.00 (dd, J = 8.2 and 14.0 Hz, 1H, $CH_aH_b$-pyr), 2.03 (br.s, 2H, $NH_2$), 1.40 (d, J = 6.9 Hz, 3H, CH(Me)(Ph)).
$^{13}C$ NMR ($CDCl_3$): δ 173.13 (C=O), 158.47, 148.88, 143.43, 136.66, 128.49, 127.06, 126.03, 124.14, 121.70

(ArC and pyrC), 55.04 (0 = C-CH-$NH_2$), 48.36 ($CH_2$-pyr), 42.20 (CH(Me)(Ph)), 22.07 (CH(Me)(Ph)).

**Claims**

1. A process for the preparation of a β-lactam compound of formula I

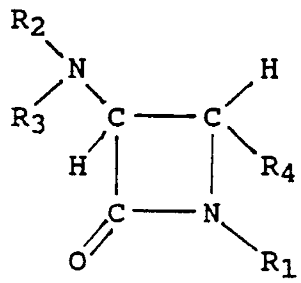

I

wherein
$R_2$ and $R_3$ are each, independently, hydrogen, or a (1-8C)alkyl, (6-18C)aryl or (1-8C)alkyl(6-18C)aryl group, each optionally substituted by a (1-8C)alkyl, (6-18C)aryl or (1-8C)alkyl(6-18C)aryl group, or the group

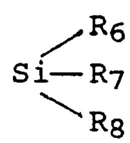

where $R_6$, $R_7$ and $R_8$ are each, independently, an optionally substituted (1-8C)alkyl, (6-18C)aryl or (1-8C)-alkyl(6-18C)aryl group,
or $R_2$ and $R_3$, together with the nitrogen atom to which they are attached, form a ring with up to 8 ring atoms, each ring atom being optionally substituted by a (1-8C)alkyl, (6-18C)aryl or (1-8C)alkyl(6-18C)aryl group;
$R_1$ is hydrogen, halogen, a (1-8C)alkyl, (2-8C)alkenyl, (1-8C)alkoxy, (6-18C)aryl, (6-18C)aryloxy, (1-8C)alkyl-(6-18C)aryl, (1-8C)alkoxy(6-18C)aryl or heterocyclic group, or is the group

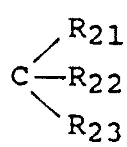

where $R_{21}$, $R_{22}$ and $R_{23}$ are each, independently, hydrogen or an optionally substituted (1-8C)alkyl, (1-8C)-alkoxy, (6-18C)aryl, (6-18C)aryloxy, (1-8C)alkyl(6-18C)aryl, (1-8C)alkoxy(6-18C)aryl, heterocyclic or

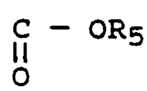

group

where $R_5$ is an (1-8C)alkyl, (6-18C)aryl or (1-8C)alkyl(6-18C)aryl group,
or is the group

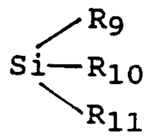

where $R_9$, $R_{10}$ and $R_{11}$ are each, independently, an optionally substituted (1-8C)alkyl, (6-18C)aryl or (1-8C)-alkyl(6-18C)aryl group;

$R_4$ is 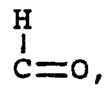, or 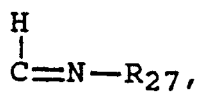,

where $R_{27}$ is an optionally substituted (1-8C)alkyl, (6-18C)aryl, (1-8C)alkyl(6-18C)aryl or heterocyclic group, or is the group

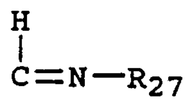

wherein $R_{27}$ is

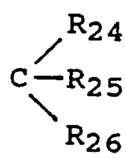

where $R_{24}$, $R_{25}$ and $R_{26}$ are each, independently, hydrogen or an optionally substituted (1-8C)alkyl, (1-8C)-alkoxy, (6-18C)aryl, (6-18C)aryloxy, (1-8C)alkyl(6-18C)aryl, (1-8C)alkoxy(6-18C)aryl or heterocyclic group, or $R_4$ is an optionally substituted heterocyclic group, which process comprises reacting a compound of formula II,

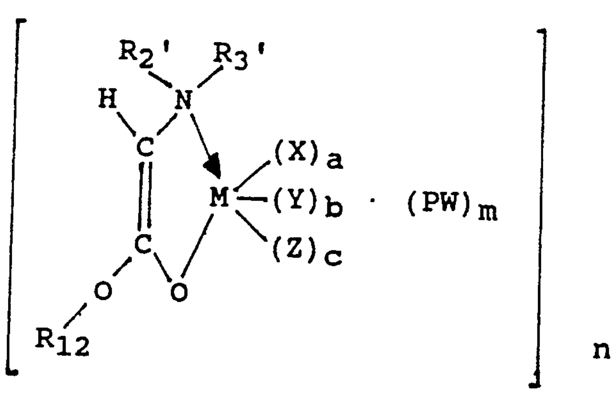

II

wherein
$R_{12}$ is a (1-8C)alkyl, (4-8C)cycloalkyl, (6-18C)aryl or (1-8C)alkyl(6-18C)aryl group, each optionally substituted by a (1-8C)alkyl, (4-8C)cycloalkyl, (6-18C)aryl or (1-8C)alkyl(6-18C)aryl group;
M is zinc, aluminum, zirconium, boron, tin or titanium;
P is an alkali metal;
X, Y and Z are each, independently, a (1-8C)alkyl or (6-18C)aryl group, or an anionic group;
W is an anionic group;
and a, b, c and m are each, independently, from 0 to 1 with the proviso that one of a, b or c is 1;
n is from 1 to 6, with a, b, c and n being integers;
$R_2'$ and $R_3'$ are as defined for $R_2$ and $R_3$, respectively, with the proviso that when $R_2$ and $R_3$ are hydrogen,

$R_2'$ and $R_3'$ form, together with the nitrogen atom to which they are attached, a ring of formula III

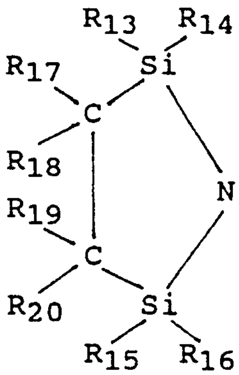

III

wherein
$R_{13}$, $R_{14}$, $R_{15}$, $R_{16}$, $R_{17}$, $R_{18}$, $R_{19}$ and $R_{20}$ are each, independently, a (1-8C)alkyl, (6-18C)aryl or (1-8C)-alkyl(6-18C)aryl group, and $R_{17}$, $R_{18}$, $R_{19}$ and $R_{20}$ are each, independently, hydrogen,
with an imine of formula IV,

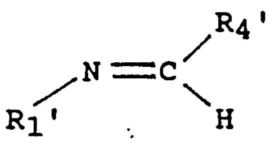

IV

where $R_1'$ is as defined for $R_1$, with the proviso that when $R_1$ is hydrogen, $R_1'$ is

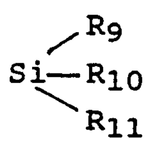

where $R_9$, $R_{10}$ and $R_{11}$ are as defined above, which group is subjected, after the reaction of compounds II and IV, to acid or base hydrolysis,
or $R_1'$ is

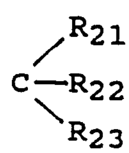

which group is subjected to the additional reaction with a solution of an alkali metal in a mixture of ammonia and a solvent,
and $R_4'$ is as defined for $R_4$, with the proviso that when

$R_4$ is 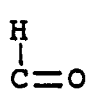, $R_4'$ is 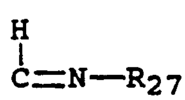

which group is subjected, after the reaction of compounds II and IV, to acid hydrolysis, or
$R_4'$ is (1,2,O-isopropylidene)ethyl, which group is subjected, after the reaction of compounds II and IV, to oxidation,
and with the additional proviso that when $R_2$ and $R_3$ are both hydrogen, the process includes the additional step of converting the ring of formula III into $NH_2$ by acid or base catalysed hydrolysis.

2. A one-pot process for the preparation of a β-lactam of formula I as defined in claim 1, which comprises reacting a glycine ester of formula V

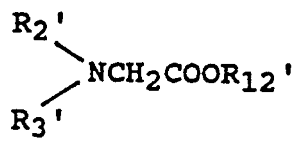

V

wherein

$R_2'$, $R_3'$ and $R_{12}'$ are as defined in claim 1,

with an imine of formula IV as defined in claim 1, in the presence of an alkali metal base and a metal compound of formula $MW(X)_a(Y)_b(Z)_c$, wherein M, W, X, Y, Z, a, b and c are as defined in claim 1.

3. A process according to any one of the preceding claims wherein W is selected from a halide, thiolate and substituted sulfonate group.

4. A process according to any one of the preceding claims wherein X, Y and Z are each, independently, an anionic group selected from halide, alkoxide, thiolate and substituted sulfonate.

5. A process according to claim 3 or 4 wherein the substituted sulfonate group is a triflate group.

6. A process according to any one of the preceding claims wherein, in formula I,

$R_2$ and $R_3$ are both the same and are selected from an ethyl and a 2,2,5,5-tetramethyl-1-aza-2,5-disilacyclopentyl group;

$R_1$ is hydrogen or a t-butyl group, and

$R_4$ is a t-butylimino, 2-pyridyl, 2-thienyl or 2-furyl group.

7. A chiral trans-β-lactam compound of formula Ia or formula Ib

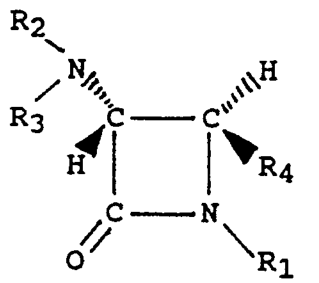

Ia

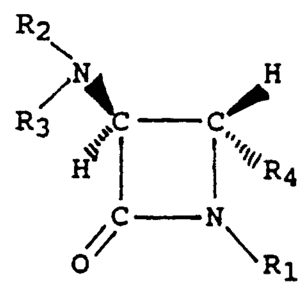

Ib

and a chiral cis-β-lactams of formula Ic and Id

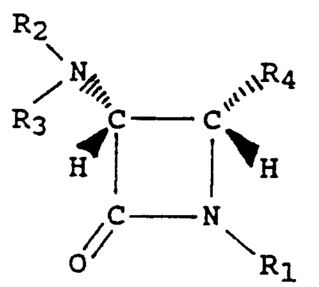

Ic

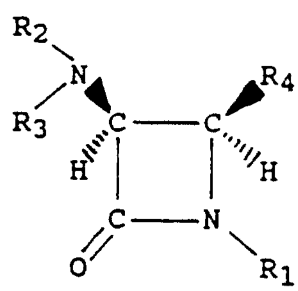

Id

wherein

$R_1$, $R_2$, $R_3$ and $R_4$ is as defined in claim 1, or

$R_2$ and $R_3$ form, together with the nitrogen atom to which they are attached, a carbamate group, with the proviso that either $R_1$ or $R_4$ comprises a chiral group or $R_1$ is hydrogen or

$$R_4 \text{ is } \overset{\displaystyle O}{\overset{\|}{C}} - H.$$

8. A compound of formula Ia, Ib, Ic or Id as indicated in claim 7 wherein
$R_2$ and $R_3$, together with the nitrogen atom to which they are attached, form a 2,2,5,5-tetramethyl-1-aza-2,5-disilacyclopentyl group a carbamate group or $R_2$ and $R_3$ are hydrogen;
$R_1$ is an α-(R)methylbenzyl or 1-p-methoxyphenyl group or hydrogen; and
$R_4$ is an α-(R)methylbenzylimino, 2-pyridyl, (1,2,O-isopropylidene)ethyl or (1-8C)alkyl group.

9. A process according to any one of the claims 1 to 6 wherein the trans-β-lactam compound is of formula Ia or formula Ib as defined in claim 7, with the provisos that when $R_2$ and $R_3$ are hydrogen, $R_2'$ and $R_3'$ can also form a carbamate group and that the process then includes the additional step of converting the carbamate group into $NH_2$ by base catalysed hydrolysis, and
$R_4$ is also defined as
(1-8C)alkyl, (1-8C)alkoxy, (6-18C)aryl, (6-18C)aryloxy, (1-8C)alkyl(6-18C)aryl, (1-8C)alkoxy(6-18C)aryl, hydroxy, sulfonyl, (2-8C)alkenyl, (2-8C)alkynyl, each optionally substituted,
or is

$$-\underset{\underset{\displaystyle O}{\|}}{C} - OR_5,$$

where $R_5$ is an (1-8C)alkyl, (6-18C)aryl or (1-8C)alkyl(6-18C)aryl group, each optionally substituted, and wherein the imine of formula IV is chiral, in a suitable, generally (weakly) polar, solvent.

10. A process according to any one of the claims 1 to 6 wherein the cis-β-lactam compound is of formula Ic or formula Id as defined in claim 7, with the provisos that when $R_2$ and $R_3$ are hydrogen, $R_2'$ and $R_3'$ can also form a carbamate group and that the process then includes the additional step of converting the carbamate group into $NH_2$ by base catalysed hydrolysis, and
$R_4$ is also defined as
(1-8C)alkyl, (1-8C)alkoxy, (6-18C)aryl, (6-18C)aryloxy, (1-8C)alkyl(6-18C)aryl, (1-8C)alkoxy(6-18C)aryl, hydroxy, sulfonyl, (2-8C)alkenyl, (2-8C)alkynyl, each optionally substituted,

$$\text{or is } -\underset{\underset{\displaystyle O}{\|}}{C} - OR_5,$$

where $R_5$ is an (1-8C)alkyl, (6-18C)aryl or (1-8C)alkyl(6-18C)aryl group, each optionally substituted, and wherein the imine of formula IV is chiral, in a suitable, generally polar, solvent.

11. A substituted amino propionic ester metal compound of formula VIa, VIb, VIc or VId,

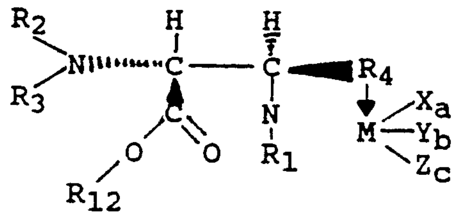

VIa

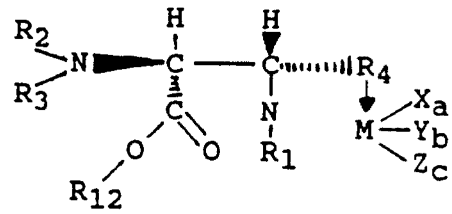

VIb

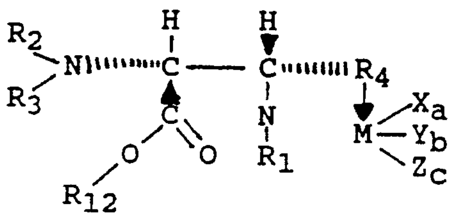

VIc

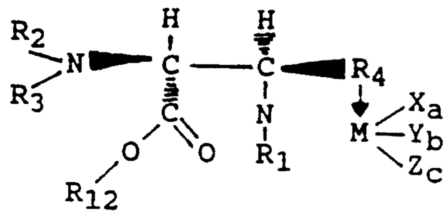

VId

wherein
$R_1$, $R_2$, $R_3$ and $R_4$ are as defined in claim 7 and $R_{12}$, M, X, Y, Z, a, b, and c are as defined in claim 1 for formula II.

12. A compound according to claim 11 wherein $R_2$ and $R_3$ are both ethyl, $R_1$ is a t-butyl group, $R_4$ is a t-butylimino group, $R_{12}$ is an ethyl group and $MW(X)_a(Y)_b(Z)_c$ is $ZnCl_2$.

13. A process for the preparation of a compound of formula VIa and VIb, or VIc and VId as defined in claim 11 which comprises reacting, in an apolar solvent, a compound of formula II as defined in claim 1 with a chiral imine of formula IV as defined in claim 9.

14. A one-pot process for the preparation of a compound of formula VIa and VIb, or VIc and VId as defined in claim 11 which comprises reacting, in an apolar solvent, a glycine ester of formula V as defined in claim 2 with a chiral imine of formula IV as defined in claim 9, in the presence of an alkali metal base and a metal compound with the formula $MW(X)_a(Y)_b(Z)_c$ where M, W, X, Y, Z, a, b and c are as defined in claim 1.

15. An enantioselective process for the preparation of a chiral trans-$\beta$-lactam of formula Ia and Ib, as defined in claim 7, which comprises adding a suitable (weakly) polar solvent to a chiral substituted amino ester metal compound of formula VIa and VIb, respectively, as defined in claim 11.

16. An enantioselective process for the preparation of a chiral cis-$\beta$-lactam of formula Ic and Id, as defined in claim 7, which comprises adding a suitable (weakly) polar solvent to a chiral substituted amino ester metal compound of formula VIc and VId, respectively, as defined in claim 11.

17. An amino acid derivative, bearing a chiral group, of formula VIIa or VIIb

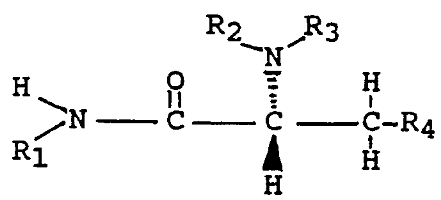

VIIa

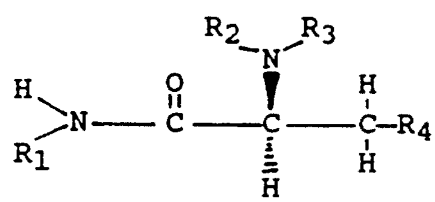

VIIb

wherein
$R_1$, $R_2$, $R_3$ and $R_4$ are as defined in claim 7 for formula Ia, Ib, Ic or Id.

18. An amino acid derivative of formula VIIa according to claim 17 wherein each of $R_2$ and $R_3$ is hydrogen, $R_1$ is a (R)$\alpha$-methylbenzyl and $R_4$ is a pyridyl group.

19. A process for the preparation of an amino acid derivative of formula VIIa or VIIb as defined in claim 17 or 18 which comprises reacting a chiral $\beta$-lactam compound of formula Ia or Ic and Ib or Id as defined in

claim 7 with hydrogen.

20. A process according to claim 19 wherein the chiral β-lactam compound of formula Ia or Ic and Ib or Id is reacted with dry ammonium formate in the presence of palladium on carbon.

21. A process according to any one of claims 1 to 6 and 9, 10, 13 and 14 wherein M in formula II is zinc.

22. A process according to claim 21 wherein the metal compound $MW(X)_a(Y)_b(Z)_c$ is $ZnCl_2$.

23. A process according to claim 2 or 14 wherein the alkali metal base is lithium diisopropylamide or sodium hexamethyldisilazane.

FIGURE 1A

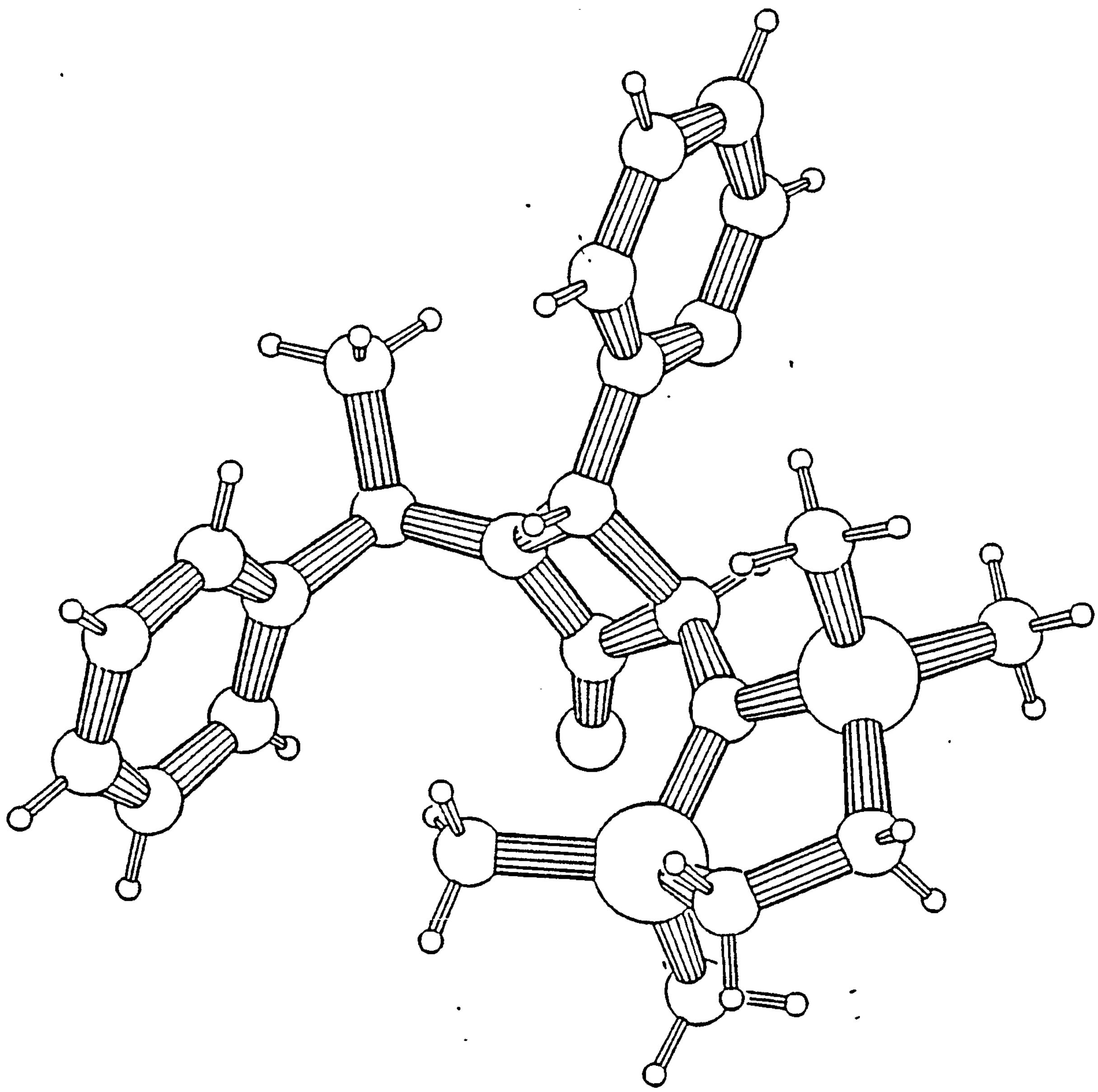

TRANS-(3R,4S)-1-(α-(R)METHYLBENZYL)-3-(2,2,5,5-TETRAMETHYL-1-AZA-2,5-DISILACYCLOPENTYL)-4-(2-PYRIDYL)-2-AZETIDINONE

FIGURE 1B

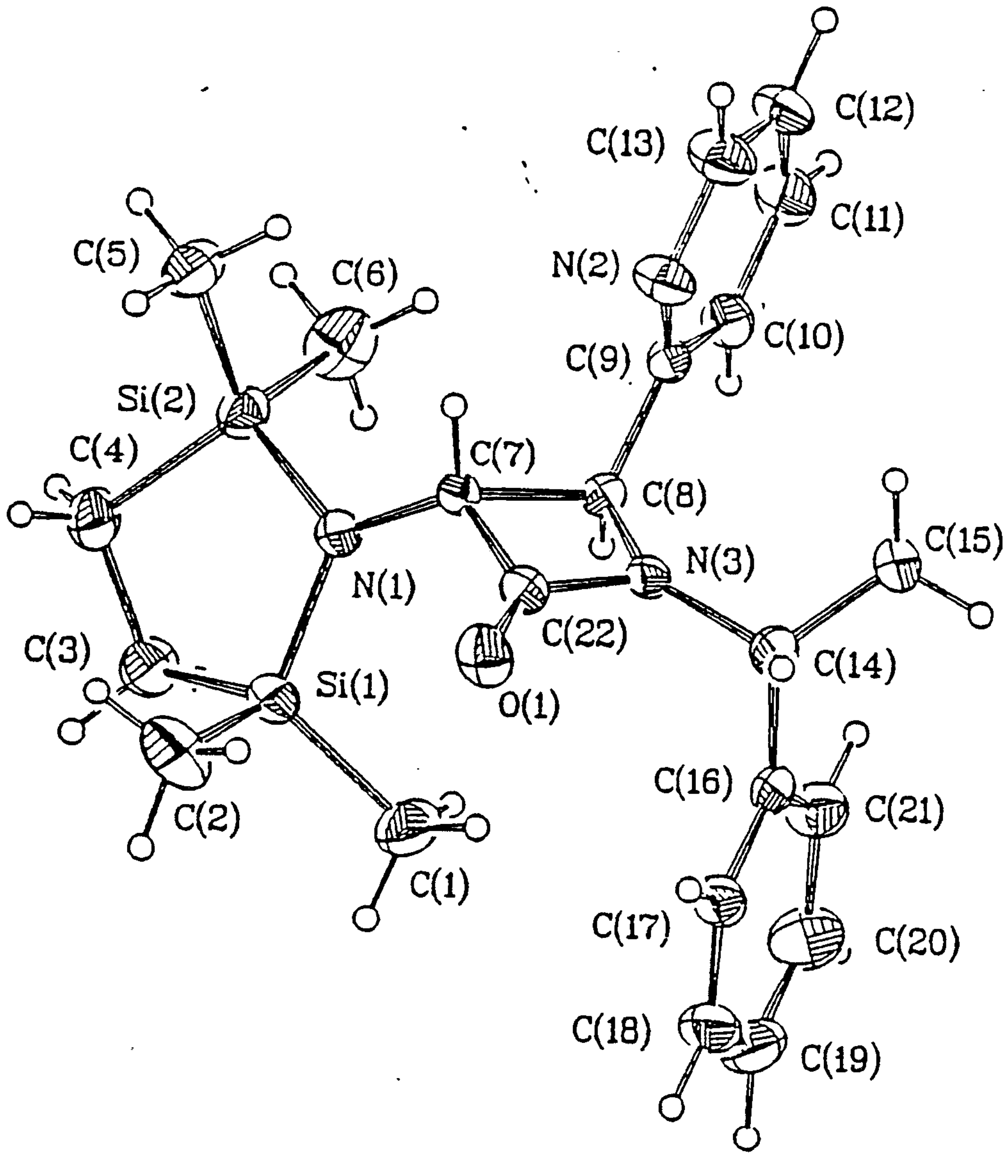

TRANS-(3R,4S)-1-(α-(R)METHYLBENZYL)-3-(2,2,5,5-TETRAMETHYL-1-AZA-2,5-DISILACYCLOPENTYL)-4-(2-PYRIDYL)-2-AZETIDINONE

European Patent Office

# EUROPEAN SEARCH REPORT

Application Number

EP 90 20 1621

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMISTRY LETTERS, vol. 7, July 1980, pages 853-856, The Chemical Society of Japan; I. OJIMA et al.: "A new and convenient route to the amides of alpha-amino acids and alpha-hydroxy acids by means of the palladium catalyzed facile cleavage of 3-substituted-4-arylazetidin-2-ones" * Whole article, especially page 854, table 1, entry 16; page 855, formula 2 * --- | 17-20 | C 07 D 205/085<br>C 07 D 401/04<br>C 07 D 409/04<br>C 07 D 405/04<br>C 07 F 7/10<br>C 07 F 3/00<br>C 07 C 251/08<br>C 07 D 213/56 |
| X | CHEMICAL ABSTRACTS, vol. 96, no. 9, 1st March 1982, page 577, abstract no. 68630t, Columbus, Ohio, US; & JP-A-81 125 348 (SAGAMI CHEMICAL RESEARCH CENTER) 01-10-1981 * Abstract * --- | 17-20 | |
| D,A | EP-A-0 281 177 (RIJKSUNIVERSITEIT UTRECHT) * Claims * --- | 1-23 | TECHNICAL FIELDS SEARCHED (Int. Cl.5) |
| A | RECUEIL DES TRAVAUX CHIMIQUES DES PAYS-BAS, vol. 106, no. 4, April 1987, pages 132-134, Royal Netherlands Chemical Society, Elsevier Science Publishers, NL; M.R.P. VAN VLIET et al.: "The unexpected formation of trans-beta-lactams in the reaction of alfa-iminoesters (R-N=C(H)C(=O)OR) with diethylzinc. Crystal structure of trans-I-(N-t-butyl)-3-(N-t-butyl-N-ethyl amino)-4-methoxycarbonylazetidin-2-one" * Whole article * --- -/- | 1-23 | C 07 D 205/00<br>C 07 D 401/00<br>C 07 D 409/00<br>C 07 D 405/00<br>C 07 F 7/00<br>C 07 F 3/00<br>C 07 C 251/00<br>C 07 D 213/00 |

The present search report has been drawn up for all claims

| Place of search | Date of completion of the search | Examiner |
|---|---|---|
| THE HAGUE | 04-09-1990 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 90 20 1621

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | TETRAHEDRON LETTERS, vol. 27, no. 15, 1986, pages 1695-1698, Pergamon Press Ltd, Oxford, GB; P. ANDREOLI et al.: "A synthetic approach to azetidinones from nitriles and lithiumtriethoxyaluminium hydride" * Whole article * --- | 1-23 | |
| A | EP-A-0 180 398 (THE REGENTS OF THE UNIVERSITY OF CALIFORNIA) * Claims * --- | 1-23 | |
| A | CHEMICAL ABSTRACTS, vol. 105, no. 25, 22nd December 1986, page 746, abstract no. 226120n, Columbus, Ohio, US; C. WANG et al.: "A stereospecific synthesis of 1-benzyl-3-amido-4-substituted phenyl-2-azetidinones", & HUAXUE XUEBAO 1986, 44(3), 250-4 ----- | 1-23 | |
| | | | TECHNICAL FIELDS SEARCHED (Int. Cl.5) |

The present search report has been drawn up for all claims

| Place of search | Date of completion of the search | Examiner |
|---|---|---|
| THE HAGUE | 04-09-1990 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)